# EUROPEAN PATENT APPLICATION

(11) **EP 3 971 903 A1**
(43) Date of publication of application: **23.03.2022**
(21) Application number: 19928338.3
(22) Date of filing: 13.05.2019
(51) Int. Cl.: G16B 30/10

(54) **EVALUATING METHOD, EVALUATING PROGRAM, AND EVALUATING DEVICE**

(71) Applicant: Fujitsu Limited, Kawasaki-shi, Kanagawa 211-8588 (JP)
(72) Inventor: KATAOKA, Masahiro, Kawasaki-shi, Kanagawa 211-8588 (JP); MATSUMURA, Ryo, Kawasaki-shi, Kanagawa 211-8588 (JP); MOGUSHI, Kaoru, Kawasaki-shi, Kanagawa 211-8588 (JP)
(74) Representative: Hoffmann Eitle
(86) International application number: PCT/JP2019/018965
(87) International publication number: WO 2020/230240

(57) **Abstract**

An evaluation device generates a new piece of base sequence data through shifting. The evaluation device specifies a partial base sequence including a base in which a genetic mutation is caused, from among a plurality of partial base sequences generated by dividing a plurality of bases included in the newly generated base sequence data from a reference position on the new piece of base sequence data according to a predetermined rule. The evaluation device performs evaluation according to an appearance state in which an arrangement of the specified partial base sequence and a partial base sequence that has a predetermined positional relationship with the specified partial base sequence from among the plurality of partial base sequences appears in the plurality of partial base sequences generated by dividing a plurality of bases included in predetermined base sequence data from the reference position on the predetermined base sequence data according to the predetermined rule.

## Description

### FIELD

The present invention relates to an evaluation method or the like.

### BACKGROUND

Genome-based drug discovery is an approaching method for developing more logically and scientifically new drugs by analyzing relationships between diseases and genomes on the basis of genome information. In this approach method, how to search for a target gene having a specific function from base sequence data of the decoded genome and link the target gene to the drug discovery is important.

At present, in addition to the base sequence data, human genome polymorphism data including single nucleotide polymorphisms (SNPs) has been steadily accumulated in database. It is possible to perform analysis called positional cloning using this database and find a target gene related to a disease for which the drug discovery is attempted.

Furthermore, genes of which expression in diseased tissues is changed from that in normal tissues are found by using a pathological model such as a human specimen or a mouse, and this is used for the genome-based drug discovery. For example, there is related art that causes a virtual mutation to normal base sequence data, evaluates a similarity with specific base sequence data of a cancer genome or the like, and estimates a possibility that the normal base sequence data becomes cancerous due to the mutation. In this related art, various mutations are caused at random positions of the normal base sequence data, and a similarity with base sequence data of various cancer genomes is evaluated.

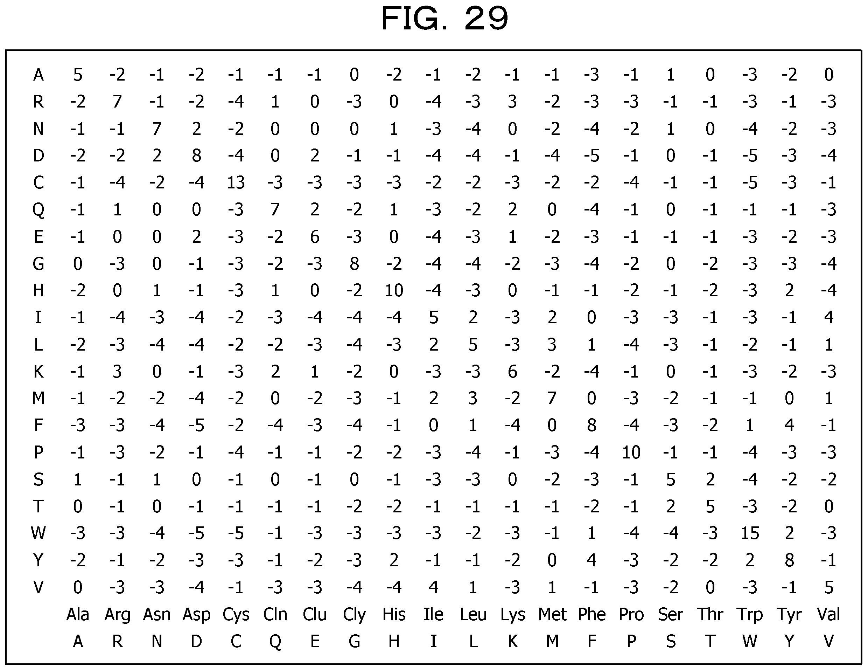

In the related art, in a case where cancers and new viruses are analyzed, FASTA and BLAST are used. In the FASTA and the BLAST, a base sequence is translated into a symbol of an amino acid, homology search is performed using an amino acid as a comparison unit, and a similarity with existing base sequence data is determined. FIG. 29 is a diagram illustrating a score matrix used for homology search.

### CITATION LIST

### [PATENT DOCUMENT]

Patent Document 1: Japanese Laid-open Patent Publication No. 2004-357702
Patent Document 2: Japanese Laid-open Patent Publication No. 2006-075162
Patent Document 3: Japanese Laid-open Patent Publication No. 2011-193868

### SUMMARY OF INVENTION

### [TECHNICAL PROBLEM]

However, with the related art described above, because various mutations are caused at the random positions of the normal base sequence data, the number of variations is large. Therefore, there is a problem in that, in a case where a similarity between base sequence data in which a mutation is caused and base sequence data of each cancer genome is comprehensively evaluated, a processing amount is large, and it takes time to perform the evaluation.

In one aspect, an object of the present invention is to provide an evaluation method, an evaluation program, and an evaluation device that can accelerate evaluation of base sequence data.

### [SOLUTION TO PROBLEM]

In a first idea, a computer executes the following processing. The computer acquires base sequence data and generates a new piece of base sequence data by shifting positions, on the base sequence data, of a plurality of bases included in the acquired base sequence data. The computer specifies a partial base sequence including a base in which it is estimated that a genetic mutation is caused, from among a plurality of partial base sequences generated by dividing the plurality of bases included in the newly generated base sequence data from a reference position on the new piece of base sequence data according to a predetermined rule. The computer evaluates the acquired base sequence data according to an appearance state in which an arrangement of the specified partial base sequence and a partial base sequence having a predetermined positional relationship with the specified partial base sequence from among the plurality of partial base sequences appears in the plurality of partial base sequences generated by dividing a plurality of bases included in predetermined base sequence data from the reference position on the predetermined base sequence data according to the predetermined rule.

According to an aspect of the embodiments, an evaluation method in which a computer executes processing includes acquiring base sequence data; generating a new piece of base sequence data by shifting positions, on the base sequence data, of a plurality of bases included in the acquired base sequence data; specifying a partial base sequence that includes a base in which it is estimated that a genetic mutation is caused, from among a plurality of partial base sequences generated by dividing the plurality of bases included in the newly generated base sequence data from a reference position on the new piece of base sequence data according to a predetermined rule; and evaluating the acquired base sequence data according to an appearance state in which an arrangement of the specified partial base sequence and a partial base sequence that has a predetermined positional relationship with the specified partial base sequence from among the plurality of partial base sequences appears in the plurality of partial base sequences generated by dividing a plurality of bases included in predetermined base sequence data from the reference position on the predetermined base sequence data according to the predetermined rule.

### [ADVANTAGEOUS EFFECTS OF INVENTION]

Evaluation of base sequence data can be accelerated.

### BRIEF DESCRIPTION OF DRAWINGS

FIG. 1 is a diagram for explaining a genome.
FIG. 2 is a diagram illustrating relationships between amino acids and bases and between amino acids and codons.
FIG. 3 is a diagram (1) for explaining processing of an evaluation device according to a first embodiment.
FIG. 4 is a diagram (2) for explaining the processing of the evaluation device according to the first embodiment.
FIG. 5 is a diagram (3) for explaining the processing of the evaluation device according to the first embodiment.
FIG. 6 is a functional block diagram illustrating a configuration of the evaluation device according to the first embodiment.
FIG. 7 is a diagram illustrating an example of a data structure of a cancer genome DB according to the first embodiment.
FIG. 8 is a diagram illustrating an example of a data structure of a conversion table according to the first embodiment.
FIG. 9 is a diagram illustrating an example of a data structure of an inverted index table according to the first embodiment.
FIG. 10 is a diagram illustrating an example of a data structure of an inverted index of a cancer genome according to the first embodiment.
FIG. 11 is a diagram for explaining an example of processing for hashing an inverted index.
FIG. 12 is a diagram for explaining an example of processing of an evaluation unit according to the first embodiment.
FIG. 13 is a diagram for explaining processing for restoring a hashed bitmap.
FIG. 14 is a flowchart illustrating a processing procedure of the evaluation device according to the first embodiment.
FIG. 15 is a diagram (1) for explaining processing of an evaluation device according to a second embodiment.
FIG. 16 is a diagram (2) for explaining the processing of the evaluation device according to the second embodiment.
FIG. 17 is a diagram (3) for explaining the processing of the evaluation device according to the second embodiment.
FIG. 18 is a diagram (4) for explaining the processing of the evaluation device according to the second embodiment.
FIG. 19 is a diagram (5) for explaining the processing of the evaluation device according to the second embodiment.
FIG. 20 is a functional block diagram illustrating a configuration of the evaluation device according to the second embodiment.
FIG. 21 is a diagram illustrating an example of a data structure of a cancer genome DB according to the second embodiment.
FIG. 22 is a diagram illustrating an example of a data structure of an inverted index table according to the second embodiment.
FIG. 23 is a diagram illustrating an example of a data structure of an inverted index of a cancer genome according to the second embodiment.
FIG. 24 is a diagram illustrating an example of a data structure of protein dictionary information according to the second embodiment.
FIG. 25 is a flowchart illustrating a processing procedure for generating an inverted index table by the evaluation device according to the second embodiment.
FIG. 26 is a flowchart (1) illustrating a processing procedure of evaluation processing executed by the evaluation device.
FIG. 27 is a flowchart (2) illustrating the processing procedure of the evaluation processing executed by the evaluation device.
FIG. 28 is a diagram illustrating an example of a hardware configuration of a computer that implements a function similar to an evaluation device according to the present embodiment.
FIG. 29 is a diagram illustrating a score matrix used for homology search.

### DESCRIPTION OF EMBODIMENTS

Hereinafter, embodiments of an evaluation method, an evaluation program, and an evaluation device disclosed herein will be described in detail with reference to the drawings. Note that the embodiments do not limit the present invention.

### [First Embodiment]

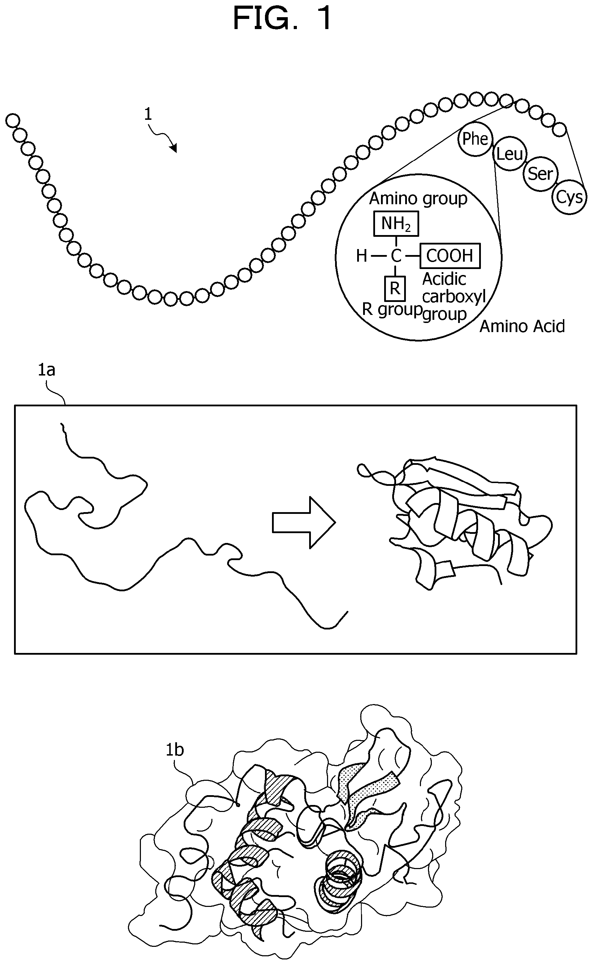

Prior to description of a first embodiment, genomes will be described. FIG. 1 is a diagram for explaining a genome. A genome 1 is genetic information in which a plurality of amino acids is linked. Here, the amino acid is determined by a plurality of bases and codons. Furthermore, the genome 1 includes a protein 1a. The protein 1a includes a chain-like linkage of 20 types of and a large number of amino acids linked to each other. Structures of the protein 1a include a primary structure, a secondary structure, and a tertiary (high-order) structure. A protein 1b is a high-order structure protein.

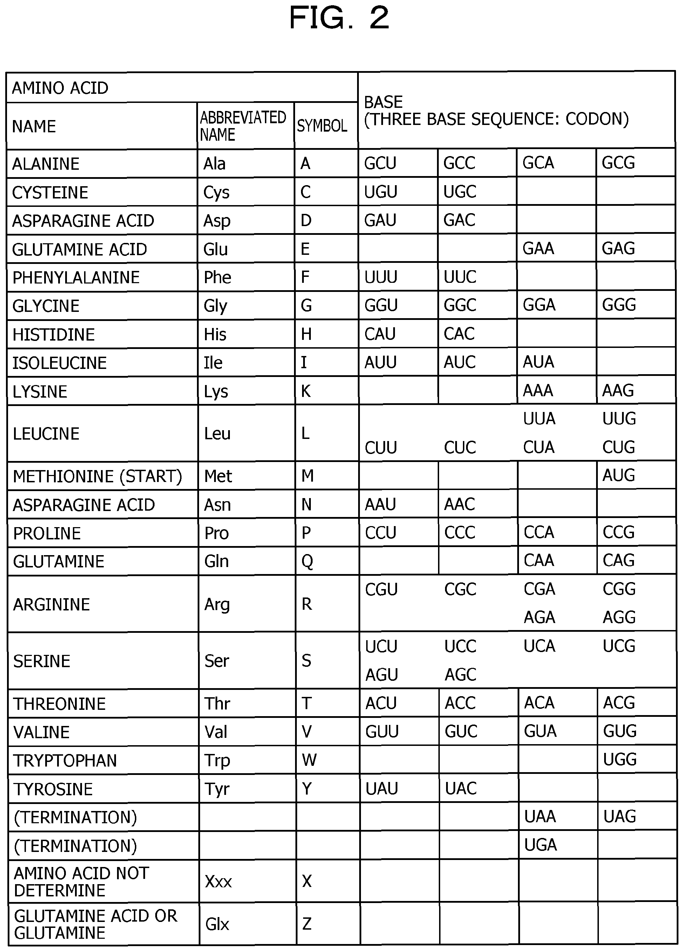

There are four types of bases in DNAs and RNAs, indicated by symbols of "A", "G", "C", and "T" or "U". Furthermore, a group of three base sequences determines each of 20 types of amino acids. Each amino acid is indicated by each of symbols of "A" to "Y". FIG. 2 is a diagram illustrating relationships between amino acids, bases, and codons. The group of three base sequences is referred to as a "codon". The sequence of the bases determines a codon, and an amino acid is determined when the codon is determined.

As illustrated in FIG. 2, a plurality of types of codons is associated with a single amino acid. Therefore, when the codon is determined, the amino acid is determined. However, if the amino acid is determined, the codon is not uniquely specified. For example, an amino acid "alanine (Ala)" is associated with codons "GCU", "GCC", "GCA" or "GCG".

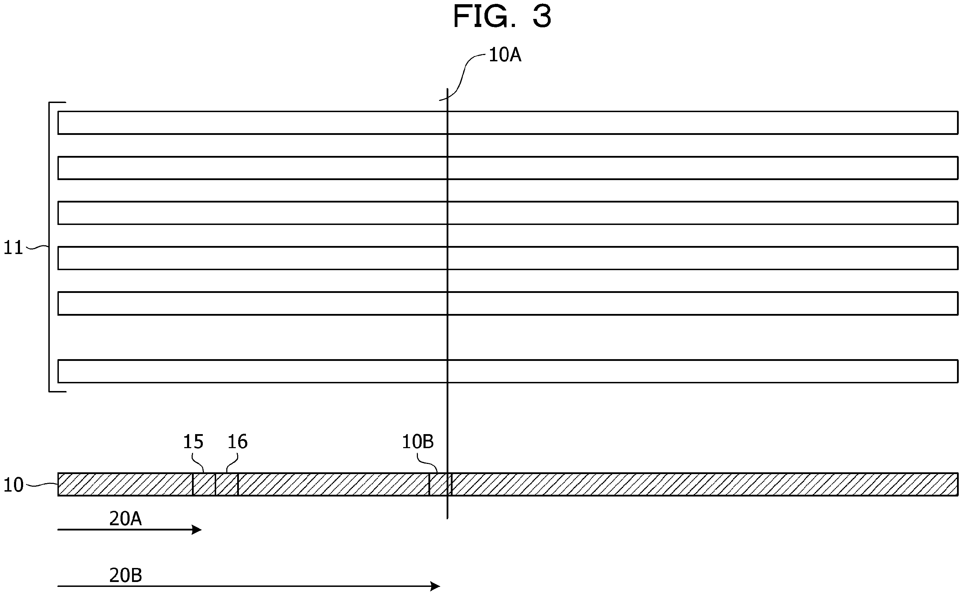

Next, an example of processing of an evaluation device according to the first embodiment will be described. FIGs. 3 to 5 are diagrams for explaining the processing of the evaluation device according to the first embodiment. First, FIG. 3 will be described. Reference genomic data 10 is whole base sequence data of a human to be a reference. Each piece of genome data 11 is base sequence data collected by a sequencer or the like from a plurality of humans. A plurality of codons (three base sequences) is arranged in the base sequence data of the reference genomic data 10 and the plurality of pieces of genome data 11. Of the three bases included in the codon, a first base from the beginning is referred to as a first base, a second base is referred to as a second base, and a third base is referred to as a third base.

The evaluation device sequentially compares the base sequence of the reference genomic data 10 with the base sequence of each piece of the genome data 11 from the beginning in codon units, and counts the number of single nucleotide polymorphism codons including a different base for each position of the codon. Because the single nucleotide polymorphism codon different from the codon of the reference genomic data 10 is inherited from a parent to a child and from a child to a grandchild, the single nucleotide polymorphism codon is referred to as a "genetic mutation". In the following description, of the base sequences of each piece of the genome data 11 at a codon position N from the beginning, the number of codons different from the codon of the reference genomic data 10 at the position N is referred to as a "mutant codon number".

The evaluation device calculates the mutant codon number for each position and specifies a position of a codon among the positions where the mutant codon number is the largest. In the following description, for evaluation of a similarity, the codon at the position where the mutant codon number is the largest is referred to as a "reference codon" for convenience. For example, in a case where the position of the codon where the mutant codon number is the largest is a position 10A, a codon 10B of the reference genomic data 10 is a reference codon.

The evaluation device specifies a codon M codons prior to the reference codon 10B as a start codon 15. Furthermore, the evaluation device selects a codon following the start codon 15 as a codon that causes a virtual mutation and specifies a mutation target codon 16. The evaluation device specifies a start codon position 20A indicating the position of the start codon 15 using the beginning as a reference on the basis of a particle size of the codon. The evaluation device specifies a reference position 20B indicating the position of the reference codon 10B using the beginning as a reference on the basis of the particle size of the codon.

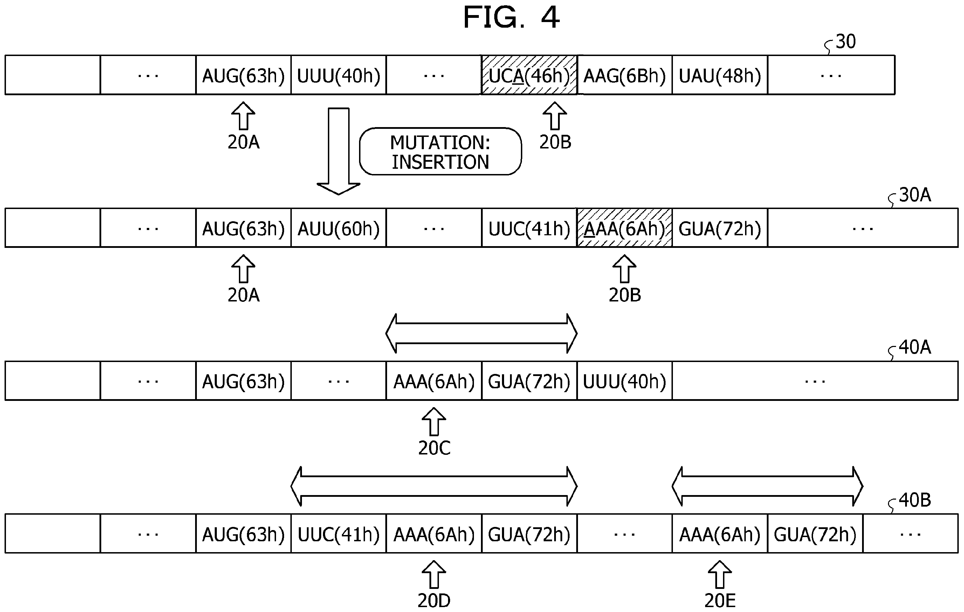

The description proceeds to FIG. 4. In FIG. 4, a case will be described where the evaluation device causes a mutation "insertion" to evaluation target genome data 30 to be evaluated. The evaluation device acquires the evaluation target genome data 30. It is assumed that the evaluation target genome data 30 be encoded in codon units. In the first embodiment, for convenience, an encoded code is indicated in parentheses next to a codon before being encoded. For example, a codon "AUG" is converted into a code "63h", and the converted code is indicated as "AUG (63h)". The reference "h" indicates a hexadecimal number.

The evaluation device specifies a start codon "AUG (63h)" on the basis of the evaluation target genome data 30 and the start codon position 20A and specifies a mutation target codon "UUU (40h)" following the start codon. The evaluation device inserts a base "A" to the beginning of the mutation target codon "UUU (40h)" and causes the mutation "insertion".

For example, the evaluation device inserts "A" into the first base of the mutation target codon, shifts the first base before insertion to the second base, and shifts the second base before insertion to the third base so as to change the mutation target codon "UUU (40h)" into "AUU (60h)". Furthermore, regarding a codon following the mutation target codon, the bases are shifted to the right. For example, for the codon following the mutation target codon, the third base of the mutation target codon is inserted into the first base of the following codon, and the subsequent base is shifted to the right. As a result, the third base "A" of the reference codon "UCA (46h)" becomes the first base of "AAA (6Ah)", and the reference position is shifted to the right.

The evaluation device generates new mutation genome data 30A by causing the mutation "insertion" to the evaluation target genome data 30. Here, although a case has been described where the base "A" is inserted into the mutation target codon, other bases "U", "G", and "C" may be inserted.

The evaluation device specifies the reference codon "AAA (6Ah)" on the basis of the mutation genome data 30A and the reference position 20B. The evaluation device compares the reference codon "AAA (6Ah)", continuous codons before and after the reference codon, and a base sequence of each cancer genome and specifies the matching codons and the length of the matching codons. Because it can be said that the longer the length of the matching codons is, the more the mutation genome data 30A is similar to the cancer genome, it can be said that the length of the matching codons (maximum length) is a "similarity".

In the following description, the reference codon and the continuous codons before and after the reference codon are referred to as a "reference codon sequence". In a case where the base sequence of the cancer genome is compared with the reference codon sequence, the evaluation device can increase a comparison speed using an inverted index of the cancer genome. The inverted index of the cancer genome is information in which an offset from the beginning of the cancer genome is associated with a type of the codon (encoded codon).

Description will be made using a cancer genome 40A. The evaluation device compares the cancer genome 40A with the reference codon "AAA (6Ah)" and specifies a position 20C of a codon same as the reference codon in the cancer genome 40A. The evaluation device compares the cancer genome 40A with the reference codon sequence using the codon at the position 20C of the cancer genome 40A as a starting point and specifies a matching codon sequence "AAA (6Ah) and GUA (72h)" and a similarity "2".

Description will be made using a cancer genome 40B. The evaluation device compares the cancer genome 40B with the reference codon "AAA (6Ah)" and specifies positions 20D and 20E of codons same as the reference codon in the cancer genome 40B. The evaluation device compares the cancer genome 40B with the reference codon sequence using the codon at the position 20D of the cancer genome 40B as a starting point and specifies a matching codon sequence "UUC (41h), AAA (6Ah), and GUA (72h)" and a similarity "3".

Furthermore, the evaluation device compares the cancer genome 40B with the reference codon sequence using the codon at the position 20E of the cancer genome 40B as a starting point and specifies a matching codon sequence "AAA (6Ah) and GUA (72h)" and a similarity "2". In a case where the plurality of codons same as the reference codon exists as in the cancer genome 40B, the evaluation device specifies the longest sequence of the codon sequences that match the reference codon sequence as a similarity of the matching codon sequence in the cancer genome. For example, the similarity of the cancer genome 40B is "3".

The evaluation device repeatedly executes the above processing on other cancer genomes so as to specify a length (similarity) of a matching codon sequence for each cancer genome. The evaluation device evaluates the cancer genome having the largest similarity as a cancer genome that is most similar to a case where the evaluation target genome data 30 becomes cancerous. Furthermore, the evaluation device sorts the plurality of cancer genomes in a descending order of the similarity and displays a list of information regarding the cancer genomes that are high on the list.

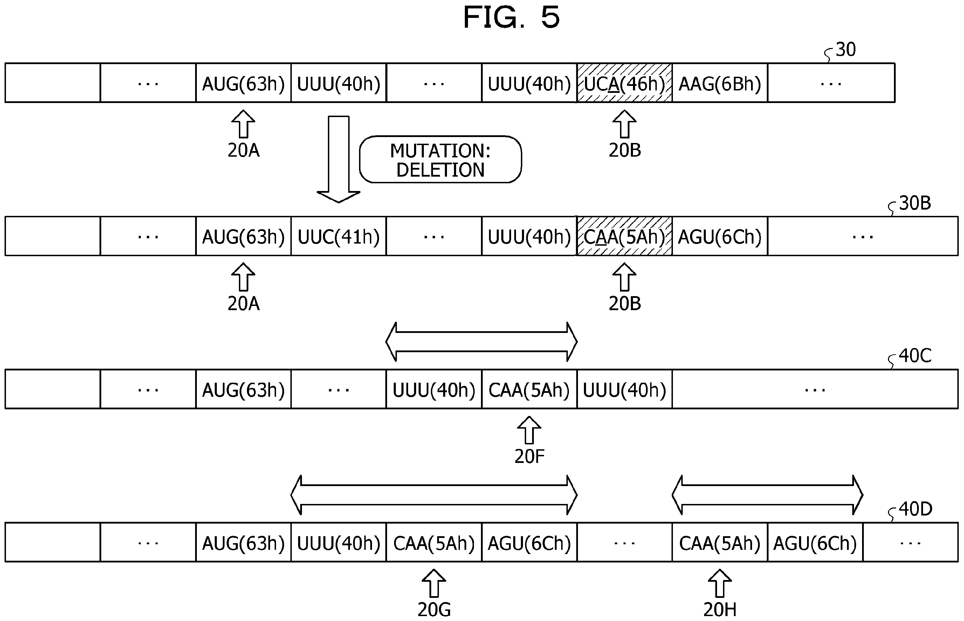

The description proceeds to FIG. 5. In FIG. 5, a case will be described where the evaluation device causes a mutation "deletion" to the normal evaluation target genome data 30. The evaluation device acquires the normal evaluation target genome data 30 to be evaluated.

The evaluation device specifies the start codon "AUG (63h)" on the basis of the evaluation target genome data 30 and the start codon position 20A and specifies the mutation target codon "UUU (40h)" following the start codon. The evaluation device removes the head base (first base) of the mutation target codon "UUU (40h)" and causes the mutation "deletion".

For example, the evaluation device deletes "U" from the first base of the mutation target codon, shifts the second base before deletion to the first base, shifts the third base before deletion to the second base, and shifts the first base of the codon following the mutation target codon to the third base of the mutation target codon so as to change the mutation target codon "UUU (40h)" into "UUC (41h)". Furthermore, regarding a codon following the mutation target codon, the bases are shifted to the left. For example, the position of the mutation target codon is set to 20A + 1, a codon at a position 20A + 2 is set as a first codon, and a codon at a position 20A + 3 is set as a second codon. A second base of the first codon is shifted to the first codon, a third base of the first codon is shifted to the second base, and a first base of the second codon is shifted to the third base of the first codon. The evaluation device shifts the following bases to the left.

The evaluation device generates new mutation genome data 30B by causing the mutation "deletion" to the normal evaluation target genome data 30.

The evaluation device specifies the reference codon "CAA (5Ah)" on the basis of the mutation genome data 30B and the reference position 20B. The evaluation device compares the reference codon "CAA (5Ah)", continuous codons before and after the reference codon (reference codon sequence), and a base sequence of each cancer genome and specifies the matching codons and the length of the matching codons.

Description will be made using a cancer genome 40C. The evaluation device compares the cancer genome 40C with the reference codon "CAA (5Ah)" and specifies a position 20F of a codon same as the reference codon in the cancer genome 40C. The evaluation device compares the cancer genome 40C with the reference codon sequence using the codon at the position 20F of the cancer genome 40C as a starting point and specifies a matching codon sequence "UUU (40h) and CAA (5Ah)" and a length "2".

Description will be made using a cancer genome 40D. The evaluation device compares the cancer genome 40D with the reference codon "CAA (5Ah)" and specifies positions 20G and 20H of codons same as the reference codon in the cancer genome 40D. The evaluation device compares the cancer genome 40D with the reference codon sequence using a codon at a position 20G of the cancer genome 40D as a starting point and specifies a matching codon sequence "UUU (40h), CAA (5Ah), and AGU (6Ch) and a length "3".

Furthermore, the evaluation device compares the cancer genome 40D with the reference codon sequence using a codon at the position 20H of the cancer genome 40D as a starting point and specifies a matching codon sequence "CAA (5Ah) and AGU (6Ch)" and a length "2". In a case where the plurality of codons same as the reference codon exists as in the cancer genome 40D, the evaluation device specifies the longest sequence of the codon sequences that match the reference codon sequence as the length of the matching codon sequence in the cancer genome. For example, regarding the cancer genome 40D, the length of the matching codon sequence is "3".

The evaluation device repeatedly executes the above processing on other cancer genomes so as to specify a length of a matching codon sequence for each cancer genome. The evaluation device evaluates the cancer genome of which the length of the matching codon sequence is the longest as a cancer genome that is most similar to a case where the evaluation target genome data 30 becomes cancerous. The evaluation device sorts the plurality of cancer genomes in a descending order of the length of the matching codon sequence and displays a list of information regarding the cancer genomes that are high on the list.

As described above, the evaluation device generates the mutation genome data 30A (30B) by causing the mutation to the mutation target codon of the evaluation target genome data 30. The evaluation device compares a reference genome sequence using a reference genome of the mutation genome data 30A (30B) as a starting point with a base sequence of a cancer genome and specifies the length (similarity) of the continuously-matching codons. The evaluation device evaluates the cancer genome of which the length of the continuously-matching codons is the longest as a cancer genome in a case where the evaluation target genome data 30 becomes cancerous.

In this way, by fixing the codon in which the mutation is caused to a codon following the start codon as a mutation target codon, the evaluation device can suppress the number of variations of genome data to be newly generated and secure the longest base sequence in which the mutation is caused. Furthermore, by narrowing the codon sequence to be compared with the cancer genome to the reference codon sequence using the reference codon as a starting point, it is possible to reduce the number of comparison trials, and it is possible to accelerate the evaluation. Furthermore, a similarity to existing cancer genomes can be evaluated on the basis of the particle size of the codon (amino acid).

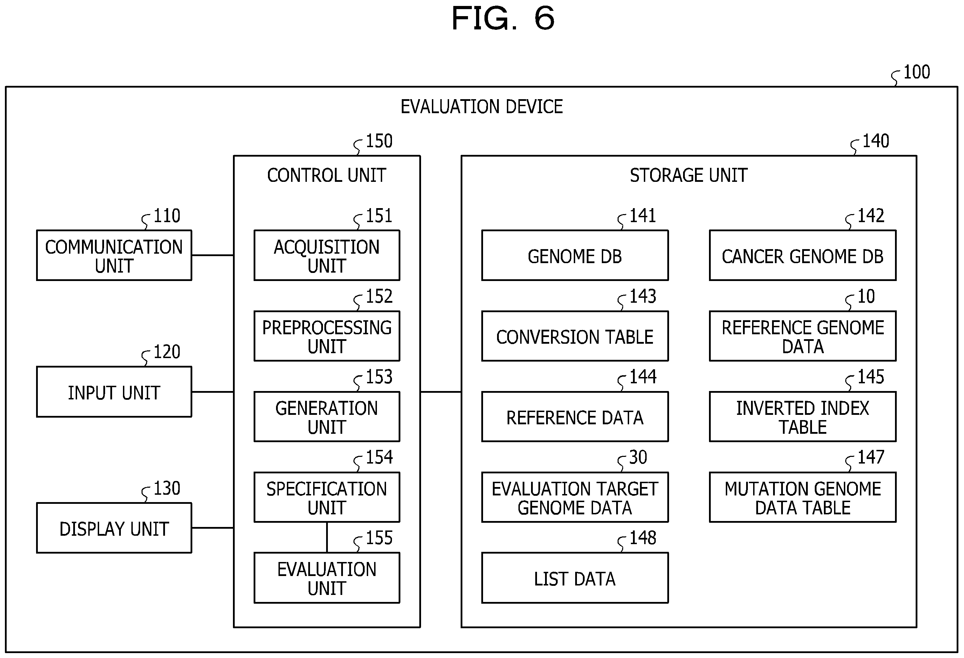

Next, an example of a configuration of the evaluation device according to the first embodiment will be described. FIG. 6 is a functional block diagram illustrating the configuration of the evaluation device according to the first embodiment. As illustrated in FIG. 6, an evaluation device 100 includes a communication unit 110, an input unit 120, a display unit 130, a storage unit 140, and a control unit 150.

The communication unit 110 is a processing unit that performs data communication with another external device (not illustrated) via a network. For example, the communication unit 110 corresponds to a communication device. For example, the communication unit 110 may receive a genome DB 141 or the like to be described later from the external device.

The input unit 120 is an input device used to input various types of information to the evaluation device 100. For example, the input unit 120 corresponds to a keyboard, a mouse, a touch panel, or the like.

The display unit 130 is a display device that displays various types of information output from the control unit 150. For example, the display unit 130 corresponds to a liquid crystal display, a touch panel, or the like.

The storage unit 140 includes the genome data base (DB) 141, a cancer genome DB 142, a conversion table 143, the reference genomic data 10, reference data 144, and an inverted index table 145. Furthermore, the storage unit 140 includes the evaluation target genome data 30, a mutation genome data table 147, and list data 148. The storage unit 140 corresponds to a semiconductor memory element such as a random access memory (RAM) or a flash memory (flash memory), or a storage device such as a hard disk drive (HDD).

The genome DB 141 is a database that holds each piece of the genome data 11 described with reference to FIG. 3. Each piece of genome data 11 is base sequence data collected by a sequencer or the like from a plurality of humans. In each piece of the genome data 11, it is assumed that codons (three base sequences) are arranged in order. Each piece of the genome data 11 may be encoded in codon units on the basis of the conversion table 143 to be described later.

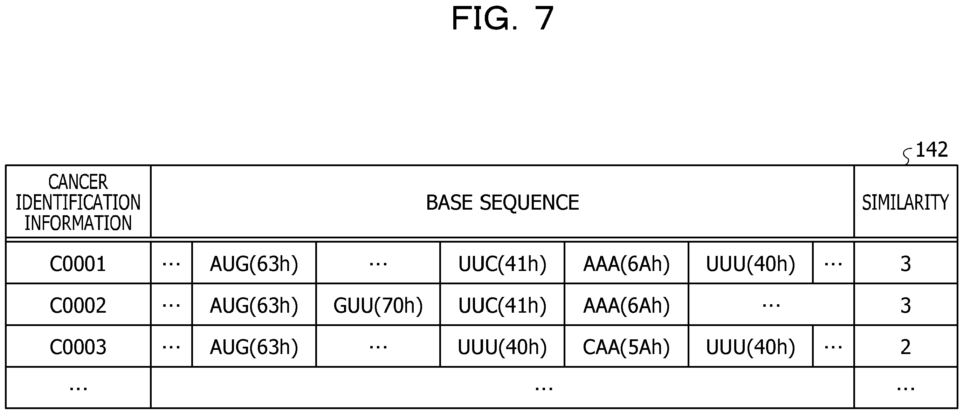

The cancer genome DB 142 is a database that holds a plurality of types of cancer genomes. FIG. 7 is a diagram illustrating an example of a data structure of a cancer genome DB according to the first embodiment. As illustrated in FIG. 7, the cancer genome DB associates identification information, a base sequence, and a similarity with each other. The cancer identification information is information that uniquely identifies a cancer genome. The base sequence is a base sequence corresponding to the cancer genome. The similarity is a value calculated by comparing the mutation genome data 30A (30B) with the cancer genome. The larger the similarity is, it is indicated that the mutation genome data 30A (30B) is more similar to the cancer genome. The base sequence of the cancer genome is encoded in codon units by a preprocessing unit 152 to be described later.

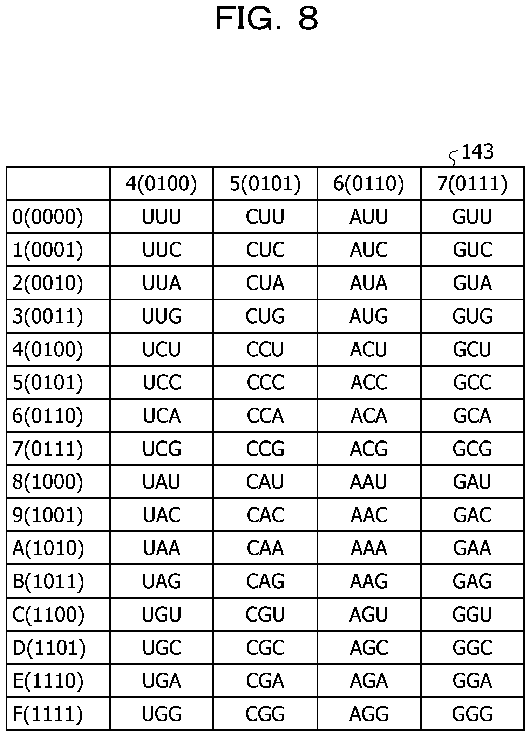

The conversion table 143 is a table that associates a codon with a code corresponding to the codon. FIG. 8 is a diagram illustrating an example of a data structure of a conversion table according to the first embodiment. As illustrated in FIG. 8, each codon is associated with each code. For example, a code of the codon "UUU" is "40h (01000000)". The reference "h" indicates a hexadecimal number.

The reference genomic data 10 is base sequence data of a preset reference (does not include genetic mutation). For example, in the reference genomic data 10, a plurality of codons is arranged.

The reference data 144 includes information regarding the "start codon position" and the "reference position" specified by the processing described with reference to FIG. 3.

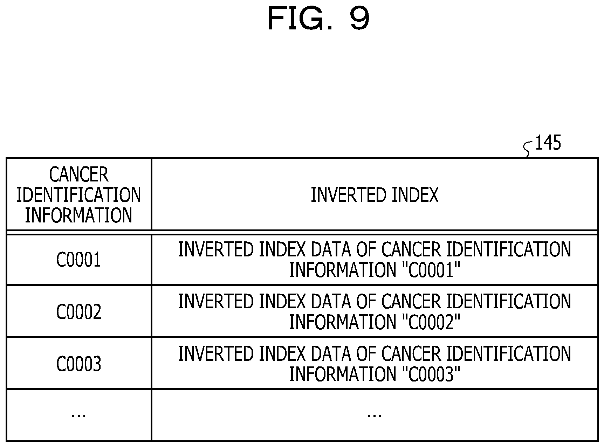

The inverted index table 145 is a table that holds each inverted index for each cancer genome. FIG. 9 is a diagram illustrating an example of a data structure of an inverted index table according to the first embodiment. The cancer identification information is information that uniquely identifies a cancer genome. The inverted index is an inverted index identified according to the cancer identification information.

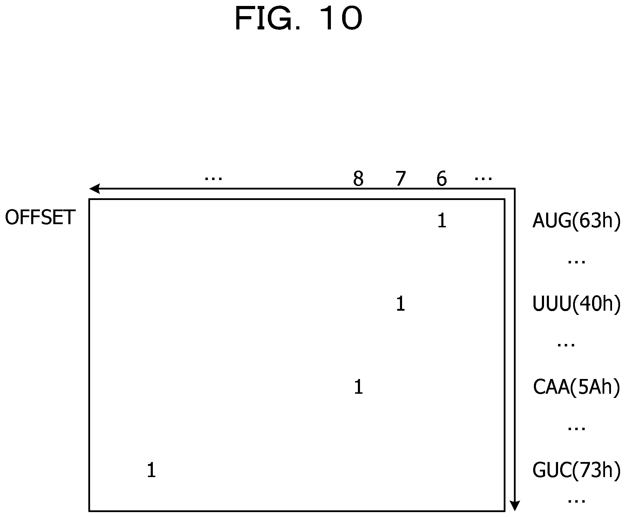

FIG. 10 is a diagram illustrating an example of a data structure of an inverted index of a cancer genome according to the first embodiment. In FIG. 10, the horizontal axis of the inverted index is an axis corresponding to an offset from the beginning of the cancer genome. The vertical axis of the inverted index is an axis corresponding to the type of the codon (codon code) The inverted index is indicated by a bitmap of "0" or "1", and all bitmaps are set to "0" in the initial state.

For example, an offset of a codon code at the beginning of the cancer genome is set to "0". In a case where a codon code "AUG (63h)" is included at the seventh position from the beginning of the cancer genome, a bit at a position where a column of an offset "6" of the inverted index intersects with a row of a codon code "AUG (63h)" is "1".

The evaluation target genome data 30 is genome data to be evaluated, collected from a human. The evaluation target genome data 30 corresponds to the evaluation target genome data 30 described with reference to FIGs. 4 and 5.

The mutation genome data table 147 is a table that holds genome data in which a mutation is caused to the evaluation target genome data 30. For example, the mutation genome data table 147 includes the mutation genome data 30A in which the mutation "insertion" is caused and the mutation genome data 30B in which the mutation "deletion" is caused.

The list data 148 is information of a list indicating a cancer genome similar to the cancer genome in a case where the evaluation target genome data 30 becomes cancerous of each cancer genome stored in the cancer genome DB 142.

The description returns to FIG. 6. The control unit 150 includes an acquisition unit 151, the preprocessing unit 152, a generation unit 153, a specification unit 154, and an evaluation unit 155. The control unit 150 can be implemented by a central processing unit (CPU), a micro processing unit (MPU), or the like. Furthermore, the control unit 150 may also be implemented by a hard wired logic such as an application specific integrated circuit (ASIC) or a field programmable gate array (FPGA).

The acquisition unit 151 is a processing unit that acquires various types of information from an external device or the like via a network. For example, in a case of acquiring the genome DB 141, the cancer genome DB 142 (before being encoded), and the reference genomic data 10, the acquisition unit 151 registers the acquired genome DB 141, cancer genome DB 142, and reference genomic data 10 to the storage unit 140.

Furthermore, the acquisition unit 151 acquires the evaluation target genome data 30 from the input unit 120 or an external device. The acquisition unit 151 registers the evaluation target genome data 30 to the storage unit 140.

The preprocessing unit 152 is a processing unit that executes various types of preprocessing. For example, the preprocessing unit 152 executes processing for generating the inverted index table 145 and processing for generating the reference data 144.

An example of the processing for generating the inverted index table 145 by the preprocessing unit 152 will be described. The preprocessing unit 152 generates an inverted index of a cancer genome while encoding the cancer genome (base sequence) registered to the cancer genome DB 142 and registers the generated inverted index to the inverted index table 145.

The preprocessing unit 152 acquires an unselected base sequence of the cancer identification information from the cancer genome DB 142 and extracts three bases from each acquired base sequence. The preprocessing unit 152 compares the extracted bases with the conversion table 143, specifies a code corresponding to the three bases (codon), and converts the three bases (codon) into the code. For example, the preprocessing unit 152 converts the codon "AUG" into a code "63h". The preprocessing unit 152 repeatedly executes the above processing so as to encode the selected base sequence in codon units.

When encoding the selected base sequence in codon units, the preprocessing unit 152 generates an inverted index corresponding to the selected base sequence. For example, the preprocessing unit 152 sets "1" to a bitmap of an inverted index corresponding to the converted codon code and an offset of the code from the beginning. The preprocessing unit 152 repeatedly executes the above processing so as to generate an inverted index corresponding to the selected base sequence. The preprocessing unit 152 registers the cancer identification information of the selected base sequence and the generated inverted index in association with each other to the inverted index table 145.

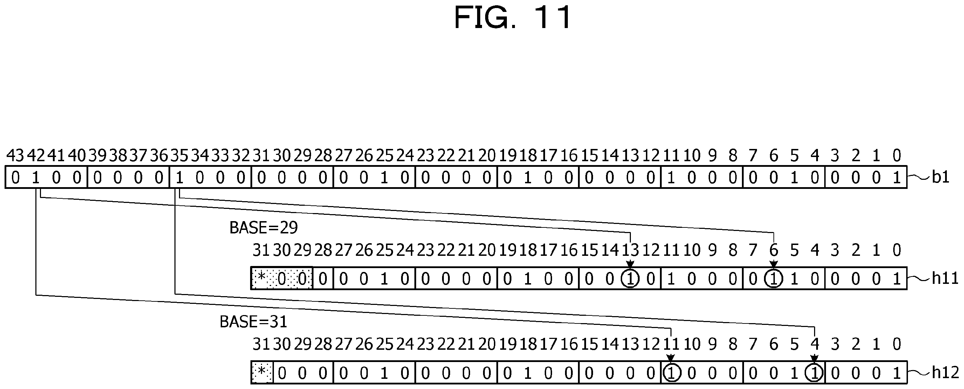

Here, when generating the inverted index, the preprocessing unit 152 may hash the inverted index to reduce an information amount. FIG. 11 is a diagram for explaining an example of processing for hashing an inverted index.

In the example described with reference to FIG. 11, a 32-bit register is assumed, and a bitmap of each row of the inverted index is hashed on the basis of prime numbers (base) "29" and "31". Here, as an example, a case will be described where hashed bitmaps h11 and h12 are generated from a bitmap b1.

It is assumed that the bitmap b1 indicate a bitmap obtained by extracting a certain row of an inverted index (for example, inverted index illustrated in FIG. 10). The hashed bitmap h11 is a bitmap hashed by a base "29". The hashed bitmap h12 is a bitmap hashed by a base "31".

The preprocessing unit 152 associates a value of a remainder obtained by dividing a position of each bit in the bitmap b1 by one base with a position in the hashed bitmap. In a case where "1" is set to a position of a bit in the corresponding bitmap b1, the preprocessing unit 152 executes processing for setting "1" to the position in the associated hashed bitmap.

An example of processing for generating the hashed bitmap h11 of the base "29" from the bitmap b1 will be described. First, the preprocessing unit 152 copies information regarding positions "0 to 28" in the bitmap b1 to the hashed bitmap h11. Subsequently, because a remainder obtained by dividing a position "35" of a bit in the bitmap b1 by a base "29" is "6", the position "35" in the bitmap b1 is associated with a position "6" in the hashed bitmap h11. Because "1" is set to the position "35" in the bitmap b1, the preprocessing unit 152 sets "1" to a position "6" in the hashed bitmap h11.

Because a remainder obtained by dividing a position "42" of a bit in the bitmap b1 by a base "29" is "13", the position "42" in the bitmap b1 is associated with a position "13" in the hashed bitmap h11. Because "1" is set to the position "42" in the bitmap b1, the preprocessing unit 152 sets "1" to the position "13" in the hashed bitmap h11.

The preprocessing unit 152 repeatedly executes the above processing regarding the positions equal to or larger than the position "29" in the bitmap b1 so as to generate the hashed bitmap h11.

An example of processing for generating the hashed bitmap h12 of the base "31" from the bitmap b1 will be described. First, the preprocessing unit 152 copies information regarding positions "0 to 30" in the bitmap b1 to the hashed bitmap h12. Subsequently, because a remainder obtained by dividing a position "35" of a bit in the bitmap b1 by a base "31" is "4", the position "35" in the bitmap b1 is associated with a position "4" in the hashed bitmap h12. Because "1" is set to the position "35" in the bitmap b1, the preprocessing unit 152 sets "1" to the position "4" in the hashed bitmap h12.

Because a remainder obtained by dividing a position "42" of a bit in the bitmap b1 by a base "31" is "11", the position "42" in the bitmap b1 is associated with a position "11" in the hashed bitmap h12. Because "1" is set to the position "42" in the bitmap b1, the preprocessing unit 152 sets "1" to the position "11" in the hashed bitmap h12.

The preprocessing unit 152 repeatedly executes the above processing regarding the positions equal to or larger than the position "31" in the bitmap b1 so as to generate the hashed bitmap h12.

The preprocessing unit 152 compresses each row of the inverted index by the above folding technique so as to hash the inverted index. Note that, to the hashed bitmaps of the bases "29" and "31", information regarding rows of the generation-source bitmap (type of encoded codon) is added. The preprocessing unit 152 repeatedly executes the above processing on the inverted index of each cancer genome and registers the hashed inverted index to the inverted index table 145.

Subsequently, an example of processing for generating the reference data 144 by the preprocessing unit 152 will be described. The processing for generating the reference data 144 by the preprocessing unit 152 corresponds to the processing described with reference to FIG. 3. The preprocessing unit 152 acquires the plurality of pieces of genome data 11 from the genome DB 141. In a case where the plurality of pieces of genome data 11 is encoded in codon units, the plurality of pieces of genome data 11 is decoded by comparing the data with the conversion table 143.

The preprocessing unit 152 compares the base of the reference genomic data 10 with the base of each piece of the genome data 11 in an order from the head base and counts the number of different bases (mutant base number) for each position of the base.

The preprocessing unit 152 calculates the mutant base number for each position, specifies a position of a base of which the mutant base number is the largest among the positions, and specifies a reference codon (for example, codon 10B in FIG. 3) including the base at the specified position. The preprocessing unit 152 specifies a codon M codons prior to the reference codon as a start codon (for example, codon 15 in FIG. 3).

The preprocessing unit 152 specifies the start codon position 20A indicating the position of the start codon 15 using the beginning of the reference genomic data 10 as a reference on the basis of the particle size of the codon. The preprocessing unit 152 specifies the reference position 20B indicating the position of the reference codon 10B using the beginning of the reference genomic data 10 as a reference on the basis of the particle size of the codon. The preprocessing unit 152 registers the information regarding the start codon position 20A and the reference position 20B to the storage unit 140 as the reference data 144.

The description returns to FIG. 6. The generation unit 153 is a processing unit that generates the mutation genome data by causing the mutations "insertion" and "deletion" to the evaluation target genome data 30. The generation unit 153 registers the generated mutation genome data to the mutation genome data table 147.

Processing for causing the mutation "insertion" by the generation unit 153 will be described with reference to FIG. 4. The generation unit 153 acquires the information regarding the start codon position 20A from the reference data 144. The generation unit 153 specifies a start codon "AUG (63h)" on the basis of the evaluation target genome data 30 and the start codon position 20A and specifies a mutation target codon "UUU (40h)" following the start codon. The generation unit 153 inserts a base "A" to the beginning of the mutation target codon "UUU (40h)" and causes the mutation "insertion".

For example, the generation unit 153 inserts "A" into the first base of the mutation target codon, shifts the first base before insertion to the second base, shifts the second base before insertion to the third base so as to change the mutation target codon "UUU (40h)" to "AUU (60h)". Furthermore, regarding a codon following the mutation target codon, the generation unit 153 shifts the bases to the right. For example, for the codon following the mutation target codon, the third base of the mutation target codon is inserted into the first base of the following codon, and the subsequent base is shifted to the right.

By causing the mutation "insertion" to the evaluation target genome data 30, the bases are shifted to the right, and the mutation genome data 30A is generated. Here, although a case has been described where the base "A" is inserted into the mutation target codon, other bases "U", "G", and "C" may be inserted.

Processing for causing the mutation "deletion" by the generation unit 153 will be described with reference to FIG. 5. The generation unit 153 specifies a start codon "AUG (63h)" on the basis of the evaluation target genome data 30 and the start codon position 20A and specifies a mutation target codon "UUU (40h)" following the start codon. The generation unit 153 removes the head base (first base) of the mutation target codon "UUU (40h)" and causes the mutation "deletion".

For example, the generation unit 153 deletes "A" from the first base of the mutation target codon, shifts the second base before deletion to the first base, shifts the third base before deletion to the second base, and shifts the first base of the codon following the mutation target codon to the third base of the mutation target codon so as to change the mutation target codon "UUU (40h)" into "UUC (41h)". Furthermore, regarding a codon following the mutation target codon, the bases are shifted to the left. For example, the position of the mutation target codon is set to 20A + 1, a codon at a position 20A + 2 is set as a first codon, and a codon at a position 20A + 3 is set as a second codon. A second base of the first codon is shifted to the first codon, a third base of the first codon is shifted to the second base, and a first base of the second codon is shifted to the third base of the first codon. The evaluation device shifts the following base to the right.

By causing the mutation "deletion" to the evaluation target genome data 30, the bases are shifted to the left, and the mutation genome data 30B is generated.

By executing the above processing, the generation unit 153 generates the mutation genome data 30A and 30B and registers the generated mutation genome data 30A and 30B to the mutation genome data table 147.

The specification unit 154 is a processing unit that specifies a reference codon sequence of the mutation genome data 30A (30B) on the basis of the mutation genome data 30A (30B) and the reference position 20B. The specification unit 154 outputs information regarding the specified reference codon sequence to the evaluation unit 155.

Processing for specifying the reference codon sequence of the mutation genome data 30A by the specification unit 154 will be described with reference to FIG. 4. The specification unit 154 acquires information regarding the reference position 20B from the reference data 144. The specification unit 154 specifies the reference codon "AAA (6Ah)" on the basis of the mutation genome data 30A and the reference position 20B. The specification unit 154 specifies a codon sequence from a reference position 20B - N_{A} to the reference position 20B + N_{A} as a reference codon sequence. It is assumed that N_{A} is a preset value.

Processing for specifying the reference codon sequence of the mutation genome data 30B by the specification unit 154 will be described with reference to FIG. 5. The specification unit 154 acquires information regarding the reference position 20B from the reference data 144. The specification unit 154 specifies the reference codon "CAA (5Ah)" on the basis of the mutation genome data 30B and the reference position 20B. The specification unit 154 specifies a codon sequence from a reference position 20B - N_{A} to the reference position 20B + N_{A} as a reference codon sequence.

The specification unit 154 outputs information regarding the reference codon and the reference codon sequence of the mutation genome data 30A and the reference codon and the reference codon sequence of the mutation genome data 30B to the evaluation unit 155.

The evaluation unit 155 is a processing unit that evaluates a similarity between each cancer genome and the mutation genome data 30A (30B) on the basis of the base sequence of the cancer genome and the mutation genome data 30A (30B). The evaluation unit 155 compares the base sequence of the cancer genome with the reference codon sequence and sets the longest length among the lengths of the matching codons as a similarity.

As described with reference to FIGs. 4 and 5, an outline of the processing of the evaluation unit 155 is to specify the longest length among the lengths of the matching codons by comparing the base sequence of the cancer genome and the reference codon sequence.

Note that the evaluation unit 155 uses the inverted index of the cancer genome in a case of specifying the length of the matching codons. In the following, an example of processing for specifying the length of the matching codons by the evaluation unit 155 using the inverted index will be described.

The evaluation unit 155 acquires an inverted index corresponding to a cancer genome to be compared with the reference codon sequence from the inverted index table 145. The evaluation unit 155 compares the reference codon (code) with the inverted index, specifies a position (offset) where a codon same as the reference codon appears on the base sequence of the cancer genome, and sets the position as a starting point in a case of the comparison.

The evaluation unit 155 specifies a code of a codon in a row corresponding to a flag "1" at positions before and after the position that is the starting point of the inverted index and specifies the length of the matching codons according to whether or not the specified code of the codon matches the code of the reference codon sequence.

Note that the evaluation unit 155 extracts a bitmap corresponding to each codon in the reference codon sequence from the inverted index and repeatedly executes shifting and AND operations so that it is possible to evaluate whether or not the codon sequence that is a part of the reference codon sequence is included in the base sequence of the cancer genome.

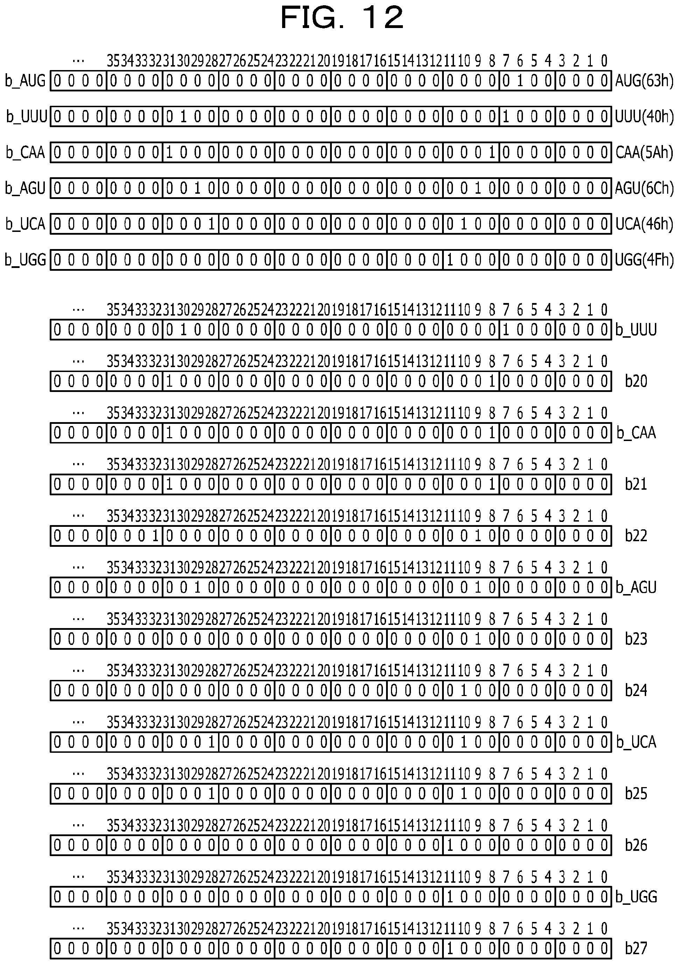

FIG. 12 is a diagram for explaining an example of processing of an evaluation unit according to the first embodiment. Here, as an example, a case will be described where the evaluation unit 155 determines whether or not there is a partial sequence "UUU (40h), CAA (5Ah), AGU (6Ch), UCA (46h), and UGG (4Fh)" including at least the reference codon of the reference codon sequence.

The evaluation unit 155 refers to the inverted index of the cancer genome to be compared and acquires a bitmap corresponding to each of the codons "UUU (40h), CAA (5Ah), AGU (6Ch), UCA (46h), and UGG (4Fh)". A bitmap of a codon code "UUU (40h)" is referred to as a bitmap b_UUU. A bitmap of a codon code "CAA (5Ah)" is referred to as a bitmap b_CAA. A bitmap of a codon code "AGU (6Ch)" is referred to as a bitmap b_AGU. A bitmap of a codon code "UCA (46h)" is referred to as a bitmap b_UCA. A bitmap of a codon code "UGG (4Fh)" is referred to as a bitmap b_UGG.

The evaluation unit 155 acquires the bitmap b_UUU and shifts the bitmap b_UUU to the left so as to generate a bitmap b20. The evaluation unit 155 acquires the bitmap b_CAA and performs an AND operation on the bitmaps b_CAA and b20 so as to generate a bitmap b21. Because "1" stands at an offset "8" of the bitmap b21, it is found that offsets 7 and 8 include codons "UUU (40h) and CAA (5Ah)".

The evaluation unit 155 generates a bitmap b22 by shifting the bitmap b21 to the left. The evaluation unit 155 acquires the bitmap b_AGU and performs an AND operation on the bitmaps b_AGU and b22 so as to generate a bitmap b23. Because "1" stands at an offset "9" of the bitmap b23, it is found that the offsets 7 to 9 include codons "UUU (40h), CAA (5Ah), and AGU (6Ch)".

The evaluation unit 155 generates a bitmap b24 by shifting the bitmap b23 to the left. The evaluation unit 155 acquires the bitmap b_UCA and performs an AND operation on the bitmaps b_UCA and b24 so as to generate a bitmap b25. Because "1" stands at an offset "10" of the bitmap b25, it is found that the offsets 7 to 10 include codons "UUU (40h), CAA (5Ah), AGU (6Ch), and UCA (46h)".

The evaluation unit 155 generates a bitmap b26 by shifting the bitmap b25 to the left. The evaluation unit 155 acquires the bitmap b_UGG and performs an AND operation on the bitmaps b_UGG and b26 so as to generate a bitmap b27. Because "1" stands at an offset "11" of the bitmap b25, it is found that the offsets 7 to 11 include codons "UUU (40h), CAA (5Ah), AGU (6Ch), UCA (46h), and UGG (4Fh)".

By executing the processing illustrated in FIG. 12, the evaluation unit 155 evaluates that the offsets "7 to 11" of the base sequence of the cancer genome to be compared include partial sequences. The evaluation unit 155 increases or reduces the number of codons in the partial sequence and repeatedly executes the above processing so as to specify the longest length (similarity) of the codons that match the base sequence of the cancer genome of the reference codon sequence.

The evaluation unit 155 evaluates the similarity with the mutation genome data 30A and the similarity with the mutation genome data 30B for the same cancer genome. The evaluation unit 155 may register each similarity to the cancer genome DB 142 or may register the larger similarity to the cancer genome DB 142.

The evaluation unit 155 registers the similarity corresponding to each piece of the cancer identification information of the cancer genome DB 142 by repeatedly executing the above processing.

The evaluation unit 155 scans each similarity in the cancer genome DB 142, specifies the cancer identification information having the largest similarity, searches the cancer genome DB 142 for the base sequence data of the cancer genome corresponding to the specified cancer identification information, and outputs the searched base sequence data of the cancer genome to the display unit 130 and displays the data on the display unit 130.

Furthermore, the evaluation unit 155 scans each similarity of the cancer genome DB 142 and sorts the pieces of cancer identification information in a descending order of the similarity. The evaluation unit 155 registers upper N_{B} pieces of the cancer identification information of the sorted pieces of cancer identification information to the list data 148. The evaluation unit 155 may search the cancer genome DB 142 for the base sequence data of the cancer genome corresponding to the upper N_{B} pieces of cancer identification information and register the searched data to the list data 148. The evaluation unit 155 may output the list data 148 to the display unit 130 and display the list data 148 on the display unit 130 or may transmit the list data 148 to an external device via a network.

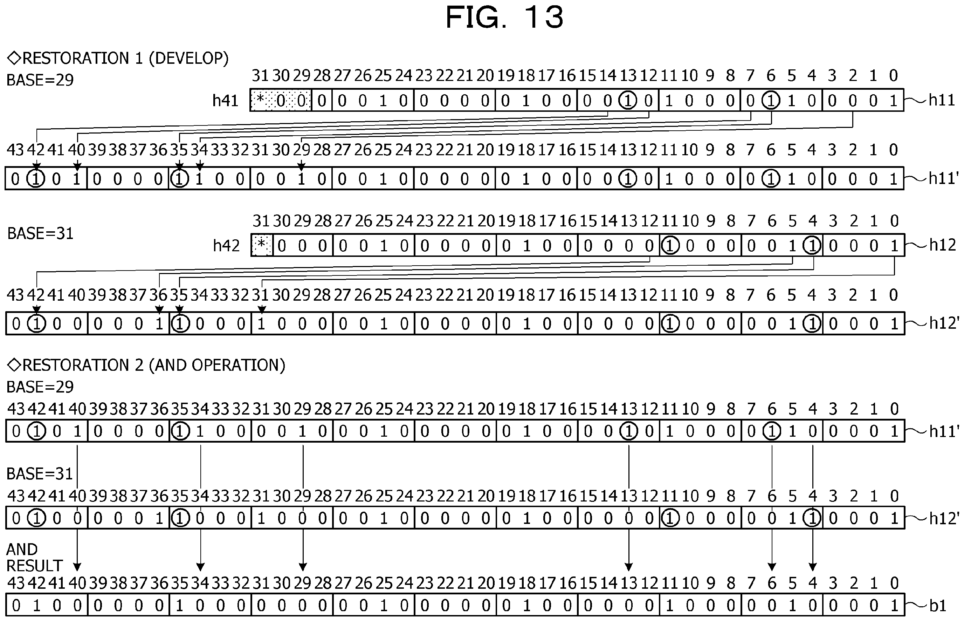

Note that, in a case where the bitmap of the inverted index is hashed, the evaluation unit 155 restores the hashed bitmap. FIG. 13 is a diagram for explaining processing for restoring the hashed bitmap. Here, as an example, a case will be described where the evaluation unit 155 restores the bitmap b1 on the basis of the hashed bitmaps h11 and h12.

The evaluation unit 155 generates an intermediate bitmap h11' from the hashed bitmap h11 of the base "29". The evaluation unit 155 copies values at positions zero to 28 in the hashed bitmap h11 to positions zero to 28, respectively, in the intermediate bitmap h11'.

Regarding values at positions 29 or subsequent values in the intermediate bitmap h11', the evaluation unit 155 repeatedly executes the processing for respectively copying the values at the positions zero to 28 in the hashed bitmap h11 for each "29". In the example illustrated in FIG. 13, an example is illustrated in which the values at the positions zero to 14 in the hashed bitmap h11 are respectively copied to the positions of the positions 29 to 43 in the intermediate bitmap h11'.

The evaluation unit 155 generates an intermediate bitmap h12' from the hashed bitmap h12 of the base "31". The evaluation unit 155 copies values at positions zero to 30 in the hashed bitmap h12 to positions zero to 30, respectively, in the intermediate bitmap h12'.

Regarding values at positions 31 or subsequent values in the intermediate bitmap h12', the evaluation unit 155 repeatedly executes the processing for respectively copying the values at the positions zero to 30 in the hashed bitmap h12 for each "31". In the example illustrated in FIG. 13, an example is illustrated in which the values at the positions zero to 12 in the hashed bitmap h12 are respectively copied to the positions of the positions 31 to 43 in the intermediate bitmap h12'.

When the intermediate bitmaps h11' and h12' are generated, the evaluation unit 155 restores the bitmap b1 before being hashed by performing an AND operation on the intermediate bitmaps h11' and h12'. Regarding other hashed bitmaps, the evaluation unit 155 can restore each bitmap corresponding to an inverted index by repeatedly executing the similar processing.

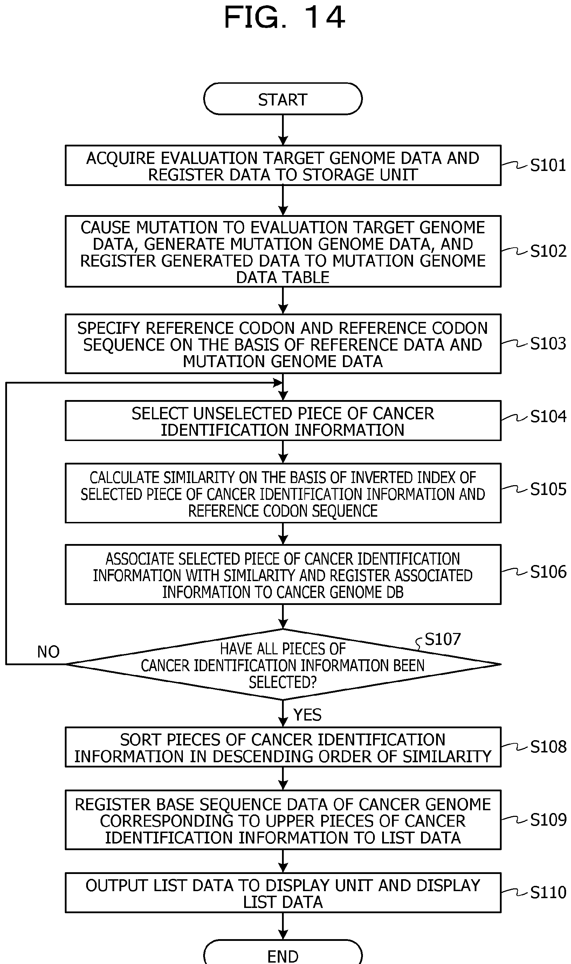

Next, an example of a processing procedure of the evaluation device 100 according to the first embodiment will be described. FIG. 14 is a flowchart illustrating a processing procedure of an evaluation device according to the first embodiment. As illustrated in FIG. 14, the acquisition unit 151 of the evaluation device 100 acquires the evaluation target genome data 30 and registers the acquired data to the storage unit 140 (step S101).

The generation unit 153 of the evaluation device 100 causes a mutation in the evaluation target genome data 30, generates the mutation genome data 30A (30B), and registers the generated data to the mutation genome data table 147 (step S102).

The specification unit 154 of the evaluation device 100 specifies the reference codon and the reference codon sequence on the basis of the reference data 144 and the mutation genome data 30A (30B) (step S103). The evaluation unit 155 of the evaluation device 100 selects an unselected piece of the cancer identification information (step S104).

The evaluation unit 155 calculates a similarity on the basis of an inverted index of the selected piece of the cancer identification information and the reference codon sequence (step S105). The evaluation unit 155 associates the selected piece of the cancer identification information with the similarity and registers the associated information to the cancer genome DB 142 (step S106).

The evaluation unit 155 determines whether or not all the pieces of the cancer identification information have been selected (step S107). In a case where all the pieces of the cancer identification information have not been selected (step S107, No), the evaluation unit 155 proceeds to step S104. In a case where all the pieces of the cancer identification information have been selected (step S107, Yes), the evaluation unit 155 proceeds to step S108.

The evaluation unit 155 sorts the pieces of the cancer identification information in a descending order of the similarity (step S108). The evaluation unit 155 registers base sequence data of a cancer genome corresponding to the upper pieces of the cancer identification information to the list data 148 (step S109). The evaluation unit 155 outputs the list data 148 to the display unit 130 and displays the list data 148 on the display unit 130 (step S110).

Next, effects of the evaluation device 100 according to the first embodiment will be described. The evaluation device 100 generates the mutation genome data 30A (30B) by causing the mutation to the mutation target codon of the evaluation target genome data 30. The evaluation device 100 compares a reference genome sequence using a reference genome of the mutation genome data 30A (30B) as a starting point with the base sequence of the cancer genome and evaluates the length (similarity) of the continuously-matching codons. The evaluation device 100 evaluates the cancer genome of which the length of the continuously-matching codons is the longest as the cancer genome in a case where the evaluation target genome data 30 becomes cancerous.

In this way, by fixing the codon in which the mutation is caused to a codon following the start codon as a mutation target codon, the evaluation device 100 can suppress the number of variations of genome data to be newly generated and secure the longest base sequence having the mutation. Furthermore, by narrowing the codon sequence to be compared with the cancer genome to the reference codon sequence using the reference codon as a starting point, it is possible to reduce the number of comparison trials, and it is possible to accelerate the evaluation. Furthermore, a similarity to existing cancer genomes can be evaluated on the basis of the particle size of the codon (amino acid). Note that there is a case where the base sequence of the cancer genome includes a receptor attached to a cell growth factor in addition to the cell growth factor in which a mutation is caused, and it is needed to remove a base sequence of the receptor.

### [Second Embodiment]

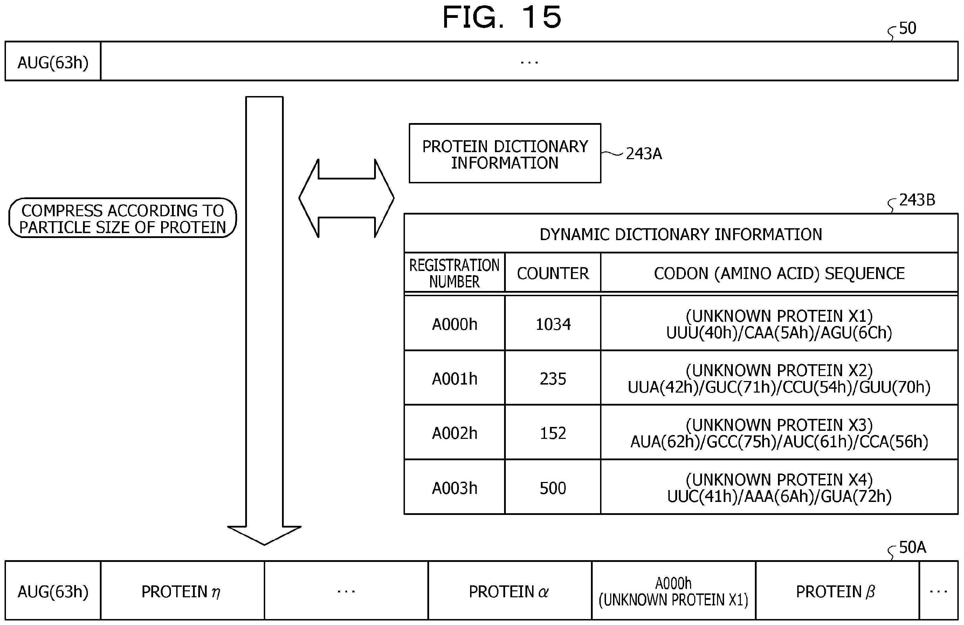

An example of processing of an evaluation device according to a second embodiment will be described. FIGs. 15 to 17 are diagrams for explaining the processing of the evaluation device according to the second embodiment. First, FIG. 15 will be described. The evaluation device acquires a cancer genome 50 encoded in codon units from a cancer genome DB. The evaluation device encodes the cancer genome 50 in protein units on the basis of protein dictionary information 243A and dynamic dictionary information 243B.

The protein dictionary information 243A is information regarding a static dictionary that associates a code of a predetermined protein (existing protein) with a code sequence in codon units. In the following description, the code sequence in codon units is referred to as a "codon (amino acid) sequence".

The dynamic dictionary information 243B is information regarding a dynamic dictionary that is used in a case where a codon sequence that is not registered in the protein dictionary information 243A is dynamically encoded to a code to a protein (unknown protein).

The evaluation device analyzes a morpheme of the cancer genome 50 in protein units so as to specify a plurality of morphemes included in the cancer genome. For example, one morpheme includes a codon sequence corresponding to a single protein.

The evaluation device compares the result of the morpheme analysis executed on the cancer genome 50 with the protein dictionary information 243A and, in a case where the morpheme hits the codon sequence of the protein dictionary information 243A, the evaluation device converts the morpheme of the cancer genome 50 into a protein code.

The evaluation device compares the result of the morpheme analysis executed on the cancer genome 50 with the protein dictionary information 243A and, in a case where the morpheme does not hit the codon sequence of the protein dictionary information 243A, the evaluation device generates a unique registration number. The evaluation device registers the registration number, an initial value "1" of a counter, and the codon sequence included in the morpheme to the dynamic dictionary information 243B and replaces the morpheme of the cancer genome 50 with the registration number so as to encode (dynamically encode) the morpheme.

Note that, in a case where the codon sequence of the morpheme that does not hit the protein dictionary information 234A has been already registered to the dynamic dictionary information 243B, the evaluation device encodes the morpheme with the corresponding registration number and adds one to the corresponding counter.

The evaluation device repeatedly performs encoding using the protein dictionary information 243A described above and dynamic encoding using the dynamic dictionary information 243B so as to generate a cancer genome 50A encoded in protein units from the cancer genome 50 encoded in codon units. Although not illustrated, the evaluation device generates an inverted index in which an offset from the beginning of the cancer genome 50 is associated with the protein code.

By repeatedly executing the above processing on each cancer genome registered to the cancer genome DB, the evaluation device registers a codon sequence of an unknown protein to the dynamic dictionary information 243B. The unknown protein that does not hit the protein dictionary information 234A is a codon sequence corresponding to a protein peculiar to cancer.

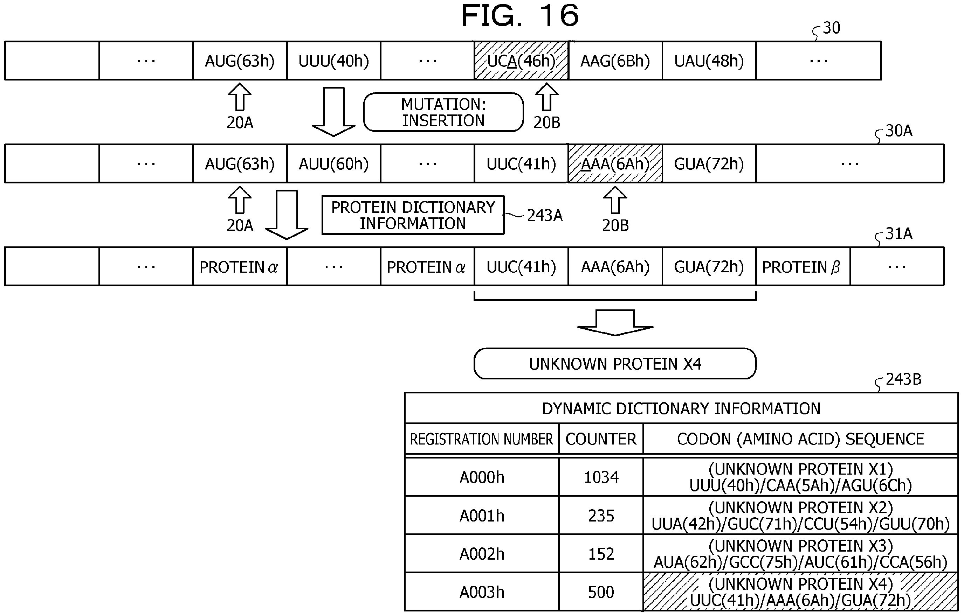

The description proceeds to FIG. 16. In FIG. 16, the evaluation device generates new mutation genome data 30A by causing a mutation "insertion" to evaluation target genome data 30 to be evaluated. Processing for causing the mutation "insertion" and generating the mutation genome data 30A is similar to the processing described with reference to FIG. 4 in the first embodiment. The evaluation device specifies a reference codon "AAA (6Ah)" of the mutation genome data 30A on the basis of a reference position 20B.

The evaluation device analyzes a morpheme in the mutation genome data 30A in protein units so as to specify a plurality of morphemes included in the mutation genome data 30A. For example, one morpheme includes a codon sequence corresponding to a protein.

The evaluation device compares the result of the morpheme analysis executed on the mutation genome data 30A with the protein dictionary information 243A and, in a case where the morpheme of the mutation genome data 30A hits the codon sequence of the protein dictionary information 243A, the evaluation device converts the morpheme of the mutation genome data 30A into a protein code. In the present embodiment, the encoded proteins are appropriately expressed by Greek letters such as proteins α, β, and γ.

In a case where the codon sequence of the morpheme including the reference codon does not hit the codon sequence of the protein dictionary information 243A, the evaluation device determines whether or not the codon sequence of the morpheme including the reference codon hits the codon sequence of the dynamic dictionary information 243B. In a case where the codon sequence of the morpheme including the reference codon does not hit the codon sequence of the dynamic dictionary information 243B, the evaluation device determines that a cancer genome similar to the mutation genome data 30A does not exist in the cancer genome DB.

On the other hand, in a case where the codon sequence of the morpheme including the reference codon hits the codon sequence of the dynamic dictionary information 243B, the evaluation device determines that the cancer genome similar to the mutation genome data 30A exists in the cancer genome DB. For example, because a codon sequence "UUC (41h)/AAA (6Ah)/GUA (72h)" including the reference codon "AAA (6Ah)" exists in the dynamic dictionary information 243B, the evaluation device determines that the cancer genome similar to the mutation genome data 30A exists in the cancer genome DB.

In a case where the codon sequence of the morpheme including the reference codon hits the codon sequence of the dynamic dictionary information 243B, the evaluation device encodes the codon sequence according to the registration number. By executing the above processing, the evaluation device generates mutation genome data 31A encoded in protein units. In the following description, in order to evaluate a similarity, an unknown protein including the reference codon, encoded into a dynamic code is referred to as a "reference protein" for convenience.

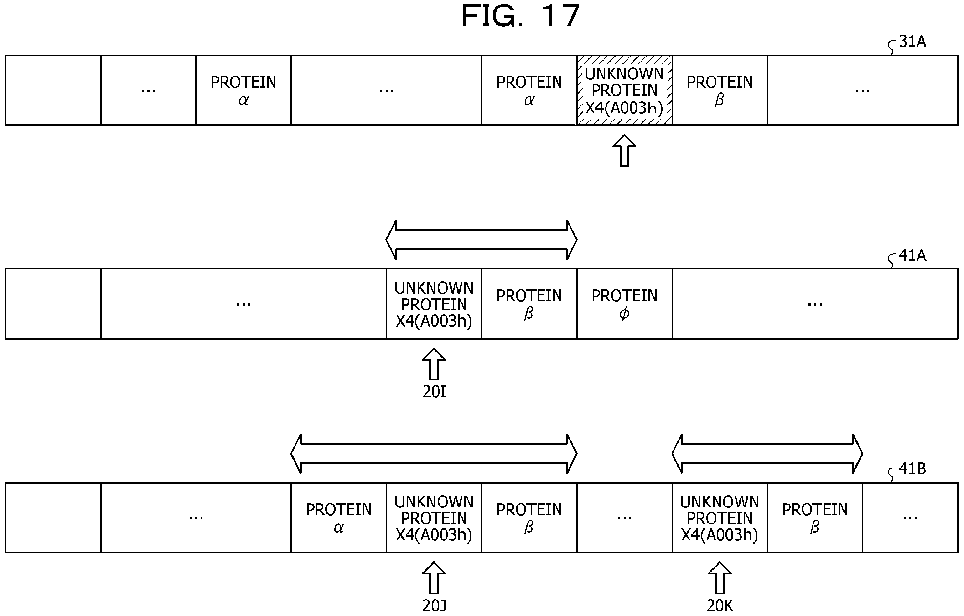

The description proceeds to FIG. 17. The evaluation device specifies a protein sequence including the reference protein and continuous proteins before and after the reference protein. In the following description, the protein sequence including the reference protein and the continuous proteins before and after the reference protein is referred to as a "reference protein sequence". The evaluation device compares the reference protein sequence with the protein sequence of the cancer genome and specifies the longest length among the length of proteins that matches the reference protein sequence as a "similarity".

Description will be made using a cancer genome 41A. The evaluation device compares the cancer genome 41A with a reference protein "unknown protein X4 (A003h)" and specifies a position 201 of a protein same as the reference protein in the cancer genome 41A. The evaluation device compares the cancer genome 41A with the reference protein sequence using the unknown protein X4 at the position 201 of the cancer genome 41A as a starting point and specifies a matching protein sequence "unknown protein X4 (A003h), protein β" and the similarity "2".

Description will be made using a cancer genome 41B. The evaluation device compares the cancer genome 41B with the reference protein "unknown protein X4 (A003h)" and specifies positions 20J and 20K of proteins same as the reference protein in the cancer genome 41B. The evaluation device compares the cancer genome 41B with the reference protein sequence using the unknown protein X4 at the position 20J of the cancer genome 41B as a starting point and specifies a matching protein sequence "protein a, unknown protein X4 (A003h), protein β" and the similarity "3".

Furthermore, the evaluation device compares the cancer genome 41B with the reference protein sequence using a protein at the position 20K of the cancer genome 41B as a starting point and specifies a matching protein sequence "unknown protein X4 (A003h), protein β" and the similarity "2". In a case where a plurality of proteins same as the reference protein exists as in the cancer genome 41B, the evaluation device specifies the longest protein sequence that matches the reference protein sequence as the similarity of the matching protein sequence in the corresponding cancer genome. For example, the similarity of the cancer genome 41B is "3".

The evaluation device repeatedly executes the above processing on other cancer genomes so as to specify a length (similarity) of a matching protein sequence for each cancer genome. The evaluation device evaluates the cancer genome having the largest similarity as a cancer genome that is most similar to a case where the evaluation target genome data 30 becomes cancerous. Furthermore, the evaluation device sorts the plurality of cancer genomes in a descending order of the similarity and displays a list of information regarding the cancer genomes that are high on the list.

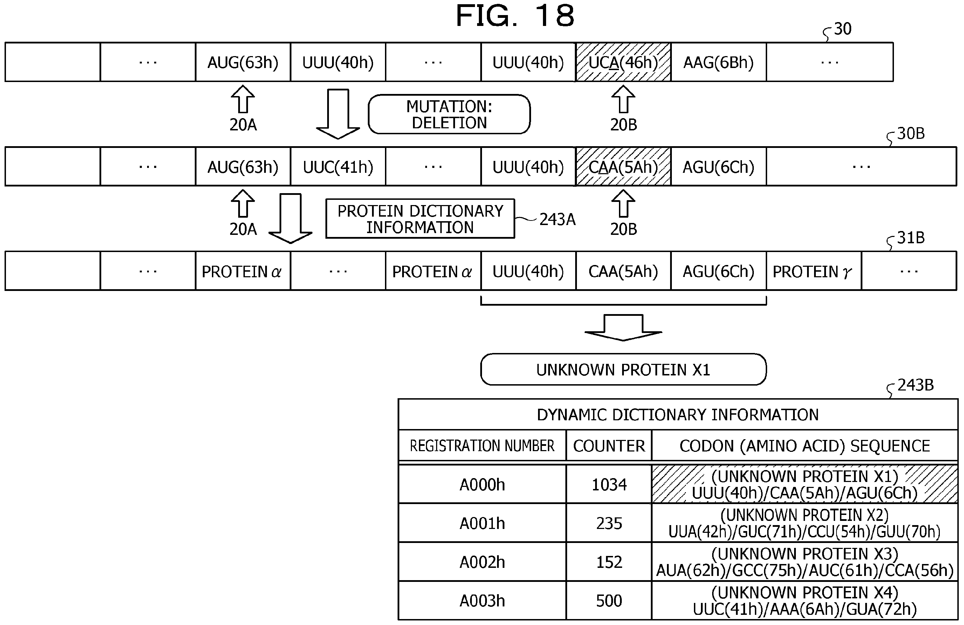

The description proceeds to FIG. 18. In FIG. 18, the evaluation device generates new mutation genome data 30B by causing a mutation "deletion" to the evaluation target genome data 30 to be evaluated. Processing for causing the mutation "deletion" and generating the mutation genome data 30B is similar to the processing described with reference to FIG. 5 in the first embodiment. The evaluation device specifies a reference codon "CAA (5Ah)" of the mutation genome data 30B on the basis of the reference position 20B.

The evaluation device analyzes a morpheme of the mutation genome data 30B in protein units so as to specify a plurality of morphemes included in the mutation genome data 30B.

The evaluation device compares the result of the morpheme analysis executed on the mutation genome data 30B with the protein dictionary information 243A and, in a case where the morpheme of the mutation genome data 30B hits the codon sequence of the protein dictionary information 243A, the evaluation device converts the morpheme of the mutation genome data 30B into a protein code.

In a case where the codon sequence of the morpheme including the reference codon does not hit the codon sequence of the protein dictionary information 243A, the evaluation device determines whether or not the codon sequence of the morpheme including the reference codon hits the codon sequence of the dynamic dictionary information 243B. In a case where the codon sequence of the morpheme including the reference codon does not hit the codon sequence of the dynamic dictionary information 243B, the evaluation device determines that a cancer genome similar to the mutation genome data 30B does not exist in the cancer genome DB.

On the other hand, in a case where the codon sequence of the morpheme including the reference codon hits the codon sequence of the dynamic dictionary information 243B, the evaluation device determines that the cancer genome similar to the mutation genome data 30A exists in the cancer genome DB. For example, because a codon sequence "UUU (40h)/CAA (5Ah)/AGU (6Ch)" including the reference codon "CAA (5Ah)" exists in the dynamic dictionary information 243B, the evaluation device determines that the cancer genome similar to the mutation genome data 30B exists in the cancer genome DB.

In a case where the codon sequence of the morpheme including the reference codon hits the codon sequence of the dynamic dictionary information 243B, the evaluation device encodes the codon sequence according to the registration number. By executing the above processing, the evaluation device generates mutation genome data 31B encoded in protein units.

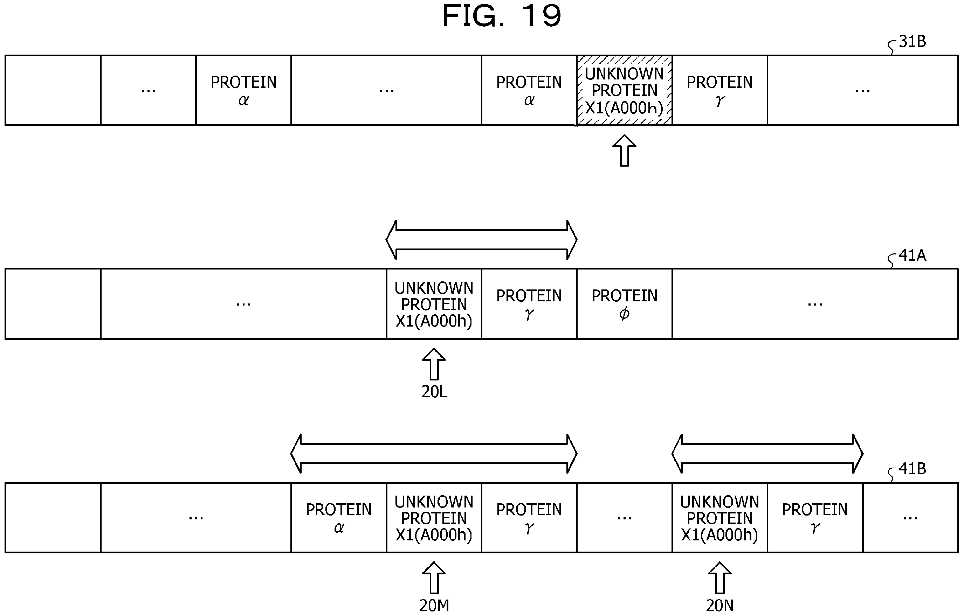

The description proceeds to FIG. 19. The evaluation device specifies a reference protein and a reference protein sequence. The evaluation device compares the reference protein sequence with the protein sequence of the cancer genome and specifies the longest length among the length of proteins that matches the reference protein sequence as a "similarity".

Description will be made using a cancer genome 41A. The evaluation device compares the cancer genome 41A with a reference protein "unknown protein X1 (A000h)" and specifies a position 20L of a protein same as the reference protein in the cancer genome 41A. The evaluation device compares the cancer genome 41A with the reference protein sequence using the unknown protein X1 at the position 20L of the cancer genome 41A as a starting point and specifies a matching protein sequence "unknown protein X1 (AOOOh), protein γ" and the similarity "2".

Description will be made using a cancer genome 41B. The evaluation device compares the cancer genome 41B with the reference protein "unknown protein X1 (A000h)" and specifies positions 20M and 20N of proteins same as the reference protein in the cancer genome 41B. The evaluation device compares the cancer genome 41B with the reference protein sequence using the unknown protein X1 at the position 20M of the cancer genome 41B as a starting point and specifies a matching protein sequence "protein a, unknown protein X1 (AOOOh), protein γ" and the similarity "3".

Furthermore, the evaluation device compares the cancer genome 41B with the reference protein sequence using a protein at the position 20N of the cancer genome 41B as a starting point and specifies a matching protein sequence "unknown protein X1 (AOOOh), protein γ" and the similarity "2". In a case where a plurality of proteins same as the reference protein exists as in the cancer genome 41B, the evaluation device specifies the longest protein sequence that matches the reference protein sequence as the similarity of the matching protein sequence in the corresponding cancer genome. For example, the similarity of the cancer genome 41B is "3".

The evaluation device repeatedly executes the above processing on other cancer genomes so as to specify a length (similarity) of a matching protein sequence for each cancer genome. The evaluation device evaluates the cancer genome having the largest similarity as a cancer genome that is most similar to a case where the evaluation target genome data 30 becomes cancerous. Furthermore, the evaluation device sorts the plurality of cancer genomes in a descending order of the similarity and displays a list of information regarding the cancer genomes that are high on the list.

As described above, the evaluation device generates the mutation genome data 30A (30B) by causing the mutation to the mutation target codon of the evaluation target genome data 30. The evaluation device compares the reference protein sequence using the reference genome of the mutation genome data 30A as a starting point with the protein sequence of the cancer genome and specifies the length (similarity) of the continuously-matching proteins. The evaluation device evaluates the cancer genome of which the length of the continuously-matching proteins is the longest as the cancer genome in a case where the evaluation target genome data 30 becomes cancerous.

In this way, by fixing the codon in which the mutation is caused to a codon following the start codon as a mutation target codon, the evaluation device can suppress the number of variations of genome data to be newly generated and secure the longest base sequence in which the mutation is caused. Furthermore, by narrowing down the protein sequence to be compared with the cancer genome to the reference protein sequence including the reference codon, it is possible to reduce the number of comparison trials, and it is possible to accelerate the evaluation. Furthermore, the similarity with the protein sequence peculiar to cancer can be evaluated on the basis of the particle size of the protein.

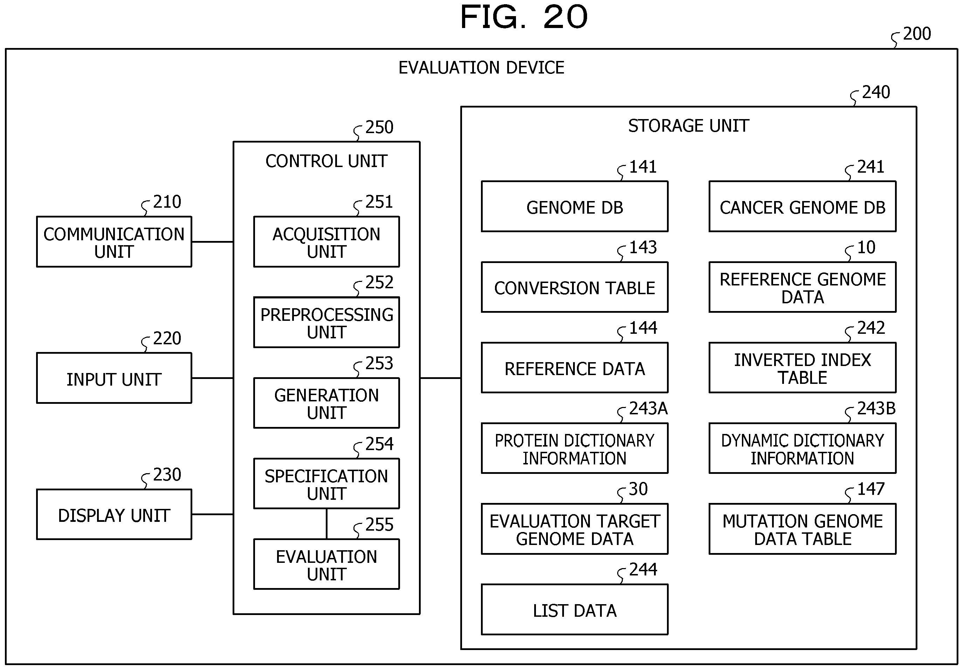

Next, an example of a configuration of the evaluation device according to the second embodiment will be described. FIG. 20 is a functional block diagram illustrating the configuration of the evaluation device according to the second embodiment. As illustrated in FIG. 20, an evaluation device 200 includes a communication unit 210, an input unit 220, a display unit 230, a storage unit 240, and a control unit 250.

The communication unit 210 is a processing unit that performs data communication with another external device (not illustrated) via a network. For example, the communication unit 210 corresponds to a communication device. For example, the communication unit 210 may receive a genome DB 141 or the like to be described later from the external device.

The input unit 220 is an input device used to input various types of information to the evaluation device 200. For example, the input unit 120 corresponds to a keyboard, a mouse, a touch panel, or the like.

The display unit 230 is a display device that displays various types of information output from the control unit 250. For example, the display unit 230 corresponds to a liquid crystal display, a touch panel, or the like.

The storage unit 240 includes the genome DB 141, a cancer genome DB 241, a conversion table 143, reference genomic data 10, reference data 144, and an inverted index table 242. Furthermore, the storage unit 240 includes the protein dictionary information 243A, the dynamic dictionary information 243B, the evaluation target genome data 30, a mutation genome data table 147, and list data 244. The storage unit 240 corresponds to a semiconductor memory element such as a RAM or a flash memory, or a storage device such as an HDD.

The genome DB 141 is a database that holds each piece of the genome data 11 described with reference to FIG. 3 in the first embodiment. Each piece of genome data 11 is base sequence data collected by a sequencer or the like from a plurality of humans. In each piece of the genome data 11, it is assumed that codons (three base sequences) are arranged in order. Each piece of the genome data 11 may be encoded in codon units on the basis of the conversion table 143.

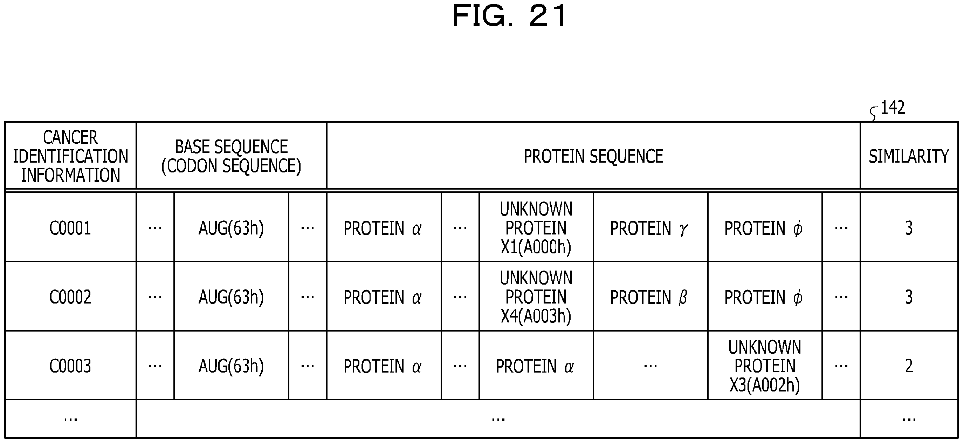

The cancer genome DB 142 is a database that holds a plurality of types of cancer genomes. FIG. 21 is a diagram illustrating an example of a data structure of a cancer genome DB according to the second embodiment. As illustrated in FIG. 21, the cancer genome DB associates identification information, a base sequence, a protein sequence, with a similarity. The cancer identification information is information that uniquely identifies a cancer genome. The base sequence is a base sequence corresponding to the cancer genome. The protein sequence is an encoded protein sequence corresponding to a cancer genome. The similarity is a value calculated by comparing the mutation genome data 30A (30B) with the cancer genome. The larger the similarity is, it is indicated that the mutation genome data 30A (30B) is more similar to the cancer genome.

The conversion table 143 is a table that associates a codon with a code corresponding to the codon. A data structure of the conversion table 143 is similar to the data structure described with reference to FIG. 8.

The reference genomic data 10 is base sequence data of a preset reference (does not include genetic mutation). For example, in the reference genomic data 10, a plurality of codons is arranged.

The reference data 144 includes information regarding the "start codon position" and the "reference position" specified by the processing described with reference to FIG. 3 in the first embodiment.

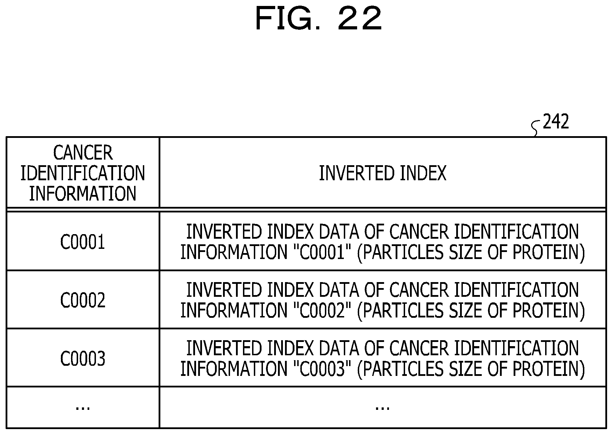

The inverted index table 242 is a table that holds each inverted index for each cancer genome. FIG. 22 is a diagram illustrating an example of a data structure of an inverted index table according to the second embodiment. The cancer identification information is information that uniquely identifies a cancer genome. The inverted index is an inverted index identified according to the cancer identification information.

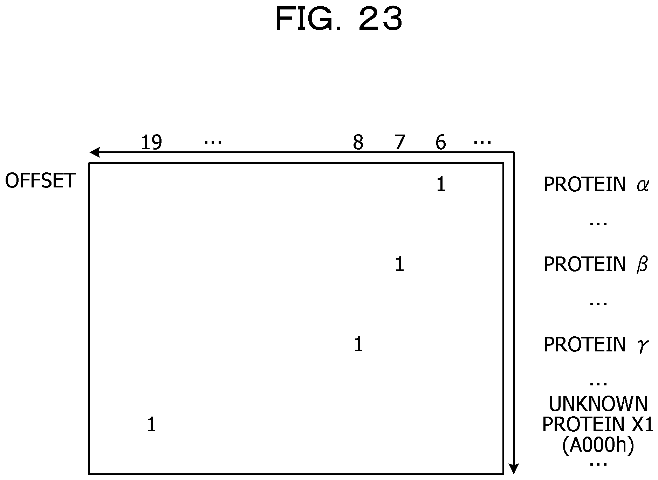

FIG. 23 is a diagram illustrating a data structure of an inverted index of a cancer genome according to the second embodiment. In FIG. 23, the horizontal axis of the inverted index is an axis corresponding to an offset from the beginning of the cancer genome. The vertical axis of the inverted index is an axis corresponding to a type of a protein (protein code). The inverted index is indicated by a bitmap of "0" or "1", and all bitmaps are set to "0" in the initial state.

For example, an offset of a codon code at the beginning of the cancer genome is set to "0". In a case where a protein code "protein a" is included at a seventh position from the beginning of the cancer genome, a bit at a position where a column of an offset "6" of the inverted index intersects with a row of the protein code "protein a" is "1". In a case where a protein code "unknown protein X1 (A000h)" is included at a 20th position from the beginning of the cancer genome, a bit at a position where a column of an offset "19" of the inverted index intersects with a row of the protein code "unknown protein X1 (A000h)" is "1".

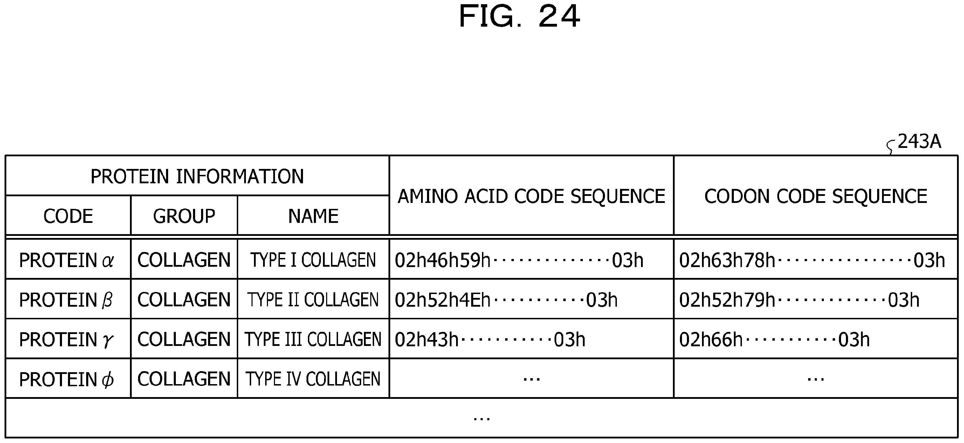

The protein dictionary information 243A is information regarding a static dictionary that associates a code of a predetermined protein (existing protein) with a code sequence in codon units. FIG. 24 is a diagram illustrating an example of a data structure of protein dictionary information according to the second embodiment. As illustrated in FIG. 24, the protein dictionary information 243A associates protein information, an amino acid code sequence, and a codon code sequence.

The protein information includes a "code" of a protein, a "group" to which the protein belongs, and a "name" of the protein. The amino acid code sequence is a sequence of amino acid codes corresponding to the protein code (type of protein). The codon code sequence is a sequence of codon codes corresponding to the protein code (type of protein).

For example, a protein "type 1 collagen" belongs to a group "collagen" and has a code "protein a". An amino acid code sequence for the code "protein a" is "02h46h59h...03h". Furthermore, a codon code sequence is "02h63h78h...03h".

The dynamic dictionary information 243B is information regarding a dynamic dictionary that is used in a case where a codon sequence that is not registered in the protein dictionary information 243A is dynamically encoded to a code to a protein (unknown protein). As described with reference to FIG. 15 or the like, the data structure of the dynamic dictionary information 243B associates a registration number, a counter, and a codon (amino acid) sequence.

The evaluation target genome data 30 is genome data to be evaluated that is designated by a user. The evaluation target genome data 30 corresponds to the evaluation target genome data 30 described with reference to FIGs. 16 and 18.

The mutation genome data table 147 is a table that holds genome data in which a mutation is caused to the evaluation target genome data 30. For example, the mutation genome data table 147 includes the mutation genome data 30A in which the mutation "insertion" is caused and the mutation genome data 30B in which the mutation "deletion" is caused.

The list data 244 is information of a list indicating a cancer genome similar to the cancer genome in a case where the evaluation target genome data 30 becomes cancerous of the cancer genomes stored in the cancer genome DB 241.

The description returns to FIG. 20. The control unit 250 includes an acquisition unit 251, a preprocessing unit 252, a generation unit 253, a specification unit 254, and an evaluation unit 255. The control unit 250 can be implemented by a CPU, an MPU, or the like. Furthermore, the control unit 250 can also be realized by a hard wired logic such as an ASIC or an FPGA.

The acquisition unit 251 is a processing unit that acquires various types of information from an external device or the like via a network. For example, in a case of acquiring the genome DB 141, the cancer genome DB 241 (before being encoded), and the reference genomic data 10, the acquisition unit 251 registers the acquired genome DB 141, cancer genome DB 241, and reference genomic data 10 to the storage unit 240. In a case of acquiring the protein dictionary information 243A, the acquisition unit 251 registers the protein dictionary information 243A to the storage unit 240.

Furthermore, the acquisition unit 251 acquires the evaluation target genome data 30 from the input unit 220 or an external device. The acquisition unit 251 registers the evaluation target genome data 30 to the storage unit 240.

The preprocessing unit 252 is a processing unit that executes various types of preprocessing. For example, the preprocessing unit 252 executes processing for generating the inverted index table 242 and processing for generating the reference data 144.

An example of the processing for generating the inverted index table 242 by the preprocessing unit 252 will be described. First, the preprocessing unit 252 compares a base sequence registered in the cancer genome DB 241 with the conversion table 143 similarly to the preprocessing unit 152 and encodes the base sequence into a codon sequence in codon units.

The preprocessing unit 252 acquires a codon sequence of unselected piece of cancer identification information from the cancer genome DB 142 and analyzes a morpheme of the codon sequence in protein units so as to specify a plurality of morphemes included in the cancer genome. For example, one morpheme includes a codon sequence corresponding to a single protein.

The preprocessing unit 252 compares the result of the morpheme analysis executed on the codon sequence with the protein dictionary information 243A and, in a case where the morpheme hits the codon sequence of the protein dictionary information 243A, the preprocessing unit 252 converts the morpheme into a protein code. Furthermore, the preprocessing unit 252 specifies an offset of a code using the beginning as a starting point in protein units, and sets "1" to a portion corresponding to the offset and the protein code in the inverted index.

The preprocessing unit 252 compares the result of the morpheme analysis executed on the codon sequence with the protein dictionary information 243A and, in a case where the morpheme does not hit the codon sequence of the protein dictionary information 243A, the evaluation device generates a unique registration number. The preprocessing unit 252 registers the registration number, an initial value "1" of a counter, and the codon sequence included in the morpheme to the dynamic dictionary information 243B and replaces the morpheme with the registration number so as to encode (dynamically encode) the morpheme. Furthermore, the preprocessing unit 252 specifies an offset of a code using the beginning as a starting point in protein units, and sets "1" to a portion corresponding to the offset and the protein code (registration number) in the inverted index.

Note that, in a case where the codon sequence of the morpheme that does not hit the protein dictionary information 234A has been already registered to the dynamic dictionary information 243B, the preprocessing unit 252 encodes the morpheme with the corresponding registration number and adds one to the corresponding counter. Furthermore, the preprocessing unit 252 specifies an offset of a code using the beginning as a starting point in protein units, and sets "1" to a portion corresponding to the offset and the protein code (registration number) in the inverted index.

By repeatedly executing the above processing on the codon sequence of each piece of the cancer identification information registered in the cancer genome DB 241, the preprocessing unit 252 registers a codon sequence of an unknown protein to the dynamic dictionary information 243B and sets "1" to the corresponding portion of the inverted index. Furthermore, the preprocessing unit 252 registers the protein sequence encoded in protein units to the cancer genome DB 241 in association with the cancer identification information.

The preprocessing unit 252 registers the inverted index corresponding to each piece of the cancer identification information generated by the above processing to the inverted index table 242. As in the first embodiment, when generating the inverted index, the preprocessing unit 252 may hash the inverted index to reduce an information amount.

Subsequently, an example of processing for generating the reference data 144 by the preprocessing unit 252 will be described. Because the processing for generating the reference data 144 by the preprocessing unit 252 is similar to the processing described with reference to FIG. 3 in the first embodiment, description thereof will be omitted. The preprocessing unit 252 registers the information regarding the start codon position 20A and the reference position 20B to the storage unit 240 as the reference data 144.

The generation unit 253 is a processing unit that generates the mutation genome data 30A (30B) by causing the mutations "insertion" and "deletion" to the evaluation target genome data 30. The generation unit 253 registers the generated mutation genome data 30A (30B) to the mutation genome data table 147. Processing for generating the mutation genome data 30A (30B) by the generation unit 253 is similar to the processing of the generation unit 153 described in the first embodiment.

The specification unit 254 specifies a reference codon of the mutation genome data 30A (30B) on the basis of the mutation genome data 30A (30B) and the reference position 20B. Furthermore, the specification unit 254 analyzes a morpheme of the mutation genome data 30A (30B) in protein units and specifies a codon sequence including the reference codon. The specification unit 254 outputs information regarding the reference codon and the codon sequence including the reference codon to the evaluation unit 255.

The evaluation unit 255 is a processing unit that evaluates a similarity between each cancer genome and the mutation genome data 30A (30B) on the basis of the protein sequence of the cancer genome and the codon sequence including the reference codon of the mutation genome data 30A (30B).

The evaluation unit 255 compares the dynamic dictionary information 243B with the codon sequence including the reference codon and determines whether or not the codon sequence including the reference codon exists in the codon sequence of the dynamic dictionary information 243B. In a case where the codon sequence including the reference codon does not exist in the codon sequence of the dynamic dictionary information 243B, the evaluation unit 255 evaluates that a cancer genome similar to the mutation genome data 30A (30B) does not exist in the cancer genome DB 241 and skips the following processing related to evaluation.

On the other hand, in a case where the codon sequence including the reference codon exists in the codon sequence of the dynamic dictionary information 243B, the evaluation unit 255 determines that the cancer genome similar to the mutation genome data 30A (30B) exists in the cancer genome DB 241 and executes the following processing related to the evaluation.

As described with reference to FIGs. 17 and 19, the evaluation unit 255 specifies the reference protein and the reference protein sequence. The evaluation unit 255 encodes the mutation genome data 30A (30B) according to the particle size of the protein on the basis of the protein dictionary information 243A and the dynamic dictionary information 243B. The evaluation unit 255 specifies a code of a protein including the reference codon, as a reference protein, for the mutation genome data 30A (30B) encoded on the basis of the particle size of the protein. The evaluation unit 255 specifies a continuous protein sequence before and after the reference protein as a "reference protein sequence".

The evaluation unit 255 compares the reference protein sequence with the protein sequence of the cancer genome and specifies the longest length among the length of proteins that matches the reference protein sequence as a "similarity".

Note that, in a case where the length of the matching proteins is specified, the evaluation unit 255 uses the inverted index of the cancer genome. In the following, an example of processing for specifying the length of the matching proteins by the evaluation unit 255 using the inverted index will be described.

The evaluation unit 255 acquires an inverted index corresponding to a cancer genome to be compared with the reference protein sequence from the inverted index table 242. The evaluation unit 255 compares the reference protein (code) with the inverted index, specifies a position (offset) where a protein same as the reference protein appears in the protein sequence of the cancer genome, and sets the position as a starting point in a case of the comparison.

The evaluation unit 255 specifies a code of a protein in a row corresponding to the flag "1" at the positions before and after the position to be the starting point of the inverted index and specifies the length of the matching codons according to whether or not the specified protein code matches the code of the reference protein sequence.

Note that the evaluation unit 255 extracts a bitmap corresponding to each protein of the reference protein sequence from the inverted index and repeatedly executes shifting and AND operations so that it is possible to evaluate whether or not a protein sequence that is a part of the reference protein sequence is included in the protein sequence of the cancer genome. Such processing corresponds to the processing for replacing the codon unit with the protein unit described with reference to FIG. 12.

The evaluation unit 255 evaluates the similarity with the mutation genome data 30A and the similarity with the mutation genome data 30B for the same cancer genome. The evaluation unit 255 may register each similarity to the cancer genome DB 241 or may register the larger similarity to the cancer genome DB 241.

The evaluation unit 255 registers the similarity corresponding to each piece of the cancer identification information of the cancer genome DB 241 by repeatedly executing the above processing.

The evaluation unit 255 scans each similarity of the cancer genome DB 241, specifies the cancer identification information having the largest similarity, searches the cancer genome DB 241 for the base sequence data of the cancer genome corresponding to the specified piece of the cancer identification information, outputs the searched protein sequence of the cancer genome to the display unit 230, and displays the protein sequence on the display unit 230.

Furthermore, the evaluation unit 255 scans each similarity of the cancer genome DB 241 and sorts the pieces of cancer identification information in a descending order of the similarity. The evaluation unit 255 registers upper N_{B} pieces of the cancer identification information of the sorted pieces of cancer identification information to the list data 244. The evaluation unit 255 may search the cancer genome DB 241 for the protein sequence of the cancer genome corresponding to the upper N_{B} pieces of cancer identification information and register the searched protein sequence to the list data 244. The evaluation unit 255 may output and display the list data 244 on the display unit 230 or may transmit the list data 244 to an external device via a network.

As in the first embodiment, in a case where the bitmap of the inverted index is hashed, the evaluation unit 255 restores the hashed bitmap.

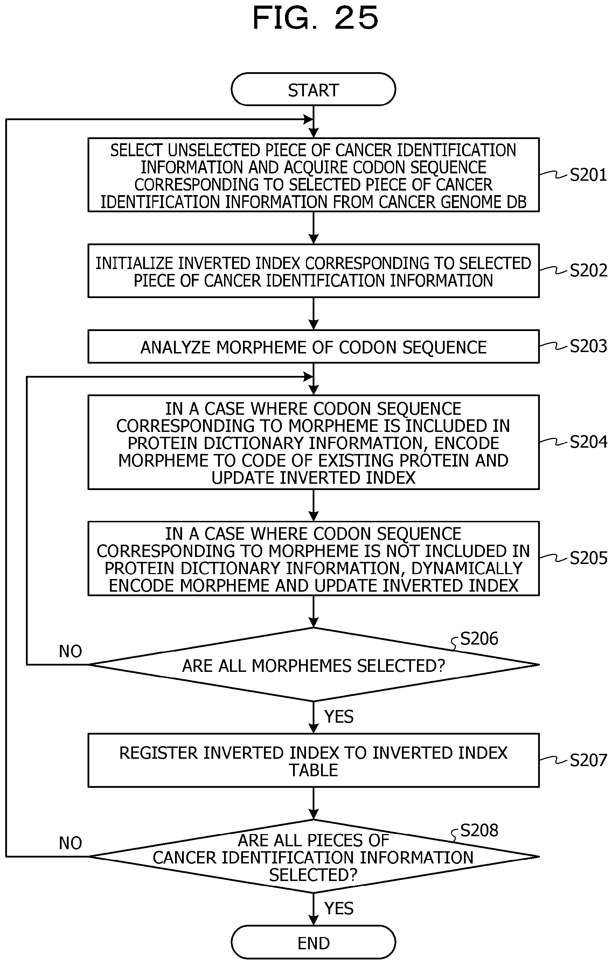

Next, an example of a processing procedure of the evaluation device 200 according to the second embodiment will be described. FIG. 25 is a flowchart illustrating a processing procedure for generating an inverted index table by the evaluation device according to the second embodiment. As illustrated in FIG. 25, the preprocessing unit 252 of the evaluation device 200 selects an unselected piece of the cancer identification information and acquires a codon sequence corresponding to the selected piece of the cancer identification information from the cancer genome DB 241 (step S201). The preprocessing unit 252 initializes an inverted index corresponding to the selected piece of the cancer identification information (step S202).

The preprocessing unit 252 analyzes a morpheme of the codon sequence (step S203). In a case where the codon sequence corresponding to the morpheme is included in the protein dictionary information 243A, the preprocessing unit 252 encodes the morpheme to a protein code of an existing protein and updates the inverted index (step S204).

In a case where the codon sequence corresponding to the morpheme is not included in the protein dictionary information 243A, the preprocessing unit 252 dynamically encodes the morpheme and updates the inverted index (step S205). In a case where all the morphemes are not selected (step S206, No), the preprocessing unit 252 proceeds to step S204.

In a case where all the morphemes are selected (step S206, Yes), the preprocessing unit 252 registers the inverted index to the inverted index table 242 (step S207). In a case where all the pieces of the cancer identification information are not selected (step S208, No), the preprocessing unit 252 proceeds to step S201. In a case where all the pieces of the cancer identification information are selected (step S208, Yes), the preprocessing unit 252 ends the processing for generating the inverted index table 242.

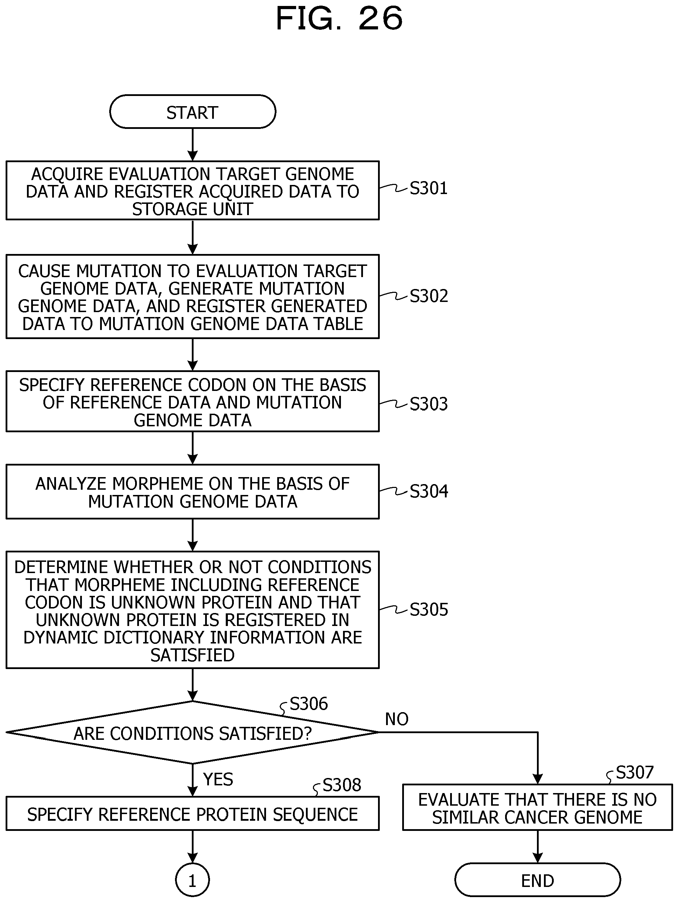

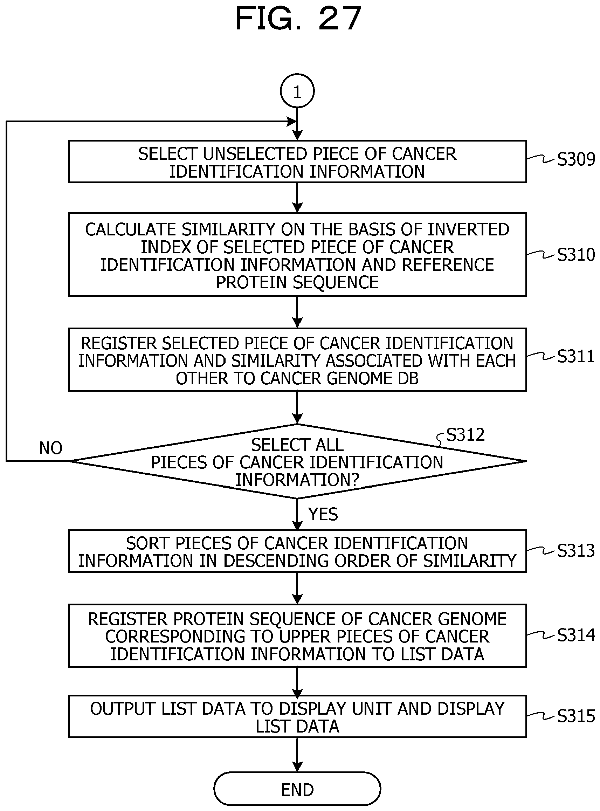

FIGs. 26 and 27 are flowcharts illustrating a processing procedure of evaluation processing executed by an evaluation device. FIG. 26 will be described. The acquisition unit 251 of the evaluation device 200 acquires the evaluation target genome data 30 and registers the acquired data to the storage unit 240 (step S301).

The generation unit 253 of the evaluation device 200 causes a mutation to the evaluation target genome data 30, generates the mutation genome data 30A (30B), and registers the generated data to the mutation genome data table 147 (step S302).

The evaluation unit 255 of the evaluation device 200 specifies a reference codon on the basis of the reference data 144 and the mutation genome data 30A (30B) (step S303). The evaluation device 200 analyzes a morpheme on the basis of the mutation genome data 30A (30B) (step S304).

The evaluation unit 255 determines whether or not the conditions that the morpheme including the reference codon is an unknown protein and the unknown protein is registered in the dynamic dictionary information 243B are both satisfied (step S305). In a case where the conditions are not satisfied (step S306, No), the evaluation unit 255 evaluates that there is no similar cancer genome (step S307).

On the other hand, in a case where the conditions are satisfied (step S306, Yes), the specification unit 254 of the evaluation device 200 specifies a reference protein sequence (step S308) and proceeds to step S309 in FIG. 27.

The description proceeds to FIG. 27. The evaluation unit 255 selects an unselected piece of the cancer identification information (step S309). The evaluation unit 255 calculates a similarity on the basis of an inverted index of the selected piece of the cancer identification information and the reference protein sequence (step S310). The evaluation unit 255 registers the selected piece of the cancer identification information and the similarity associated with each other to the cancer genome DB 241 (step S311).

In a case where all the pieces of the cancer identification information have not been selected (step S312, No), the evaluation unit 255 proceeds to step S309. The evaluation unit 255 sorts the pieces of the cancer identification information in a descending order of the similarity (step S313).

The evaluation unit 255 registers a protein sequence of the cancer genome corresponding to the upper pieces of the cancer identification information to the list data 244 (step S314). The evaluation unit 255 outputs the list data 244 to the display unit 230 and displays the list data 244 on the display unit 230 (step S315).

Next, effects of the evaluation device 200 according to the second embodiment will be described. The evaluation device 200 generates the mutation genome data 30A (30B) by causing the mutation to the mutation target codon of the evaluation target genome data 30. The evaluation device 200 compares the reference protein sequence using the reference genome of the mutation genome data 30A (30B) as a starting point with the protein sequence of the cancer genome and specifies the length (similarity) of the continuously-matching proteins. The evaluation device 200 evaluates the cancer genome of which the length of the continuously-matching proteins is the longest as the cancer genome in a case where the evaluation target genome data 30 becomes cancerous.

In this way, because the evaluation device 200 fixes a codon in which a mutation is caused as a mutation target codon, the evaluation device 200 can suppress the number of variations of newly generated genome data. Furthermore, by narrowing down the protein sequence to be compared with the cancer genome to the reference protein sequence including the reference codon, it is possible to reduce the number of comparison trials, and it is possible to accelerate the evaluation. Furthermore, the similarity with the protein sequence peculiar to cancer can be evaluated on the basis of the particle size of the protein.

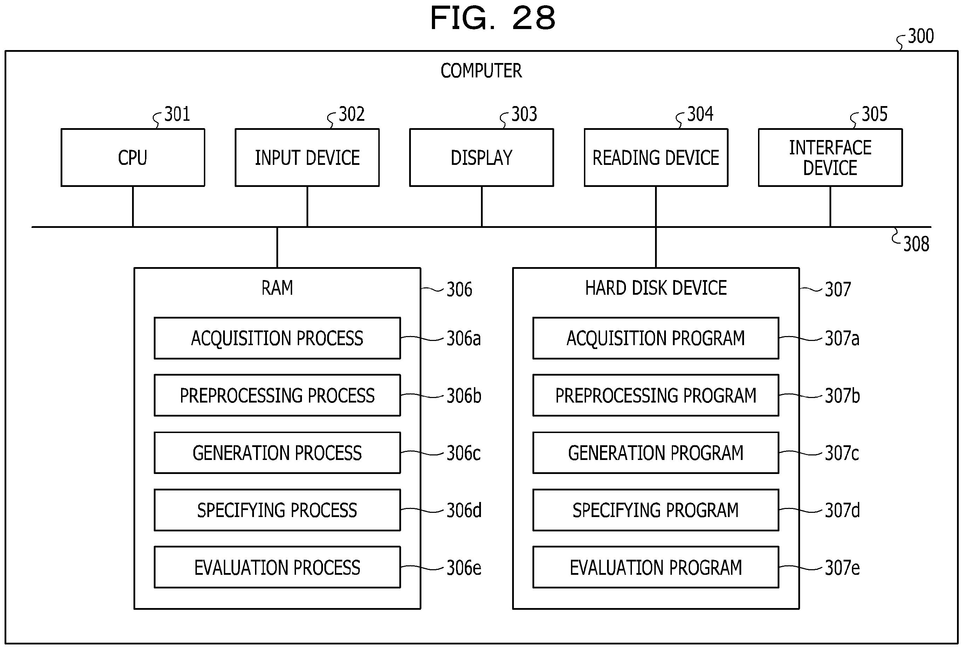

Next, an example of a hardware configuration of a computer that implements a function similar to the evaluation device 200 (100) described in the present embodiment will be described. FIG. 28 is a diagram illustrating an example of a hardware configuration of a computer that implements a function similar to an evaluation device according to the present embodiment.

As illustrated in FIG. 28, a computer 300 includes a CPU 301 that executes various types of calculation processing, an input device 302 that accepts data input from a user, and a display 303. Furthermore, the computer 300 includes a reading device 304 that reads a program and the like from a storage medium, and an interface device 305 that exchanges data with an external device or the like via a wired or wireless network. The computer 300 includes a RAM 306 that temporarily stores various types of information, and a hard disk device 307. Then, each of the devices 301 to 307 is connected to a bus 308.

The hard disk device 307 includes an acquisition program 307a, a preprocessing program 307b, a generation program 307c, a specifying program 307d, and an evaluation program 307e. The CPU 301 reads the acquisition program 307a, the preprocessing program 307b, the generation program 307c, the specifying program 307d, and the evaluation program 307e and develops the programs on the RAM 306.

The acquisition program 307a functions as an acquisition process 306a. The preprocessing program 307b functions as a preprocessing process 306b. The generation program 307c functions as a generation process 306c. The specifying program 307d functions as a specifying process 306d. The evaluation program 307e functions as an evaluation process 306e.

Processing of the acquisition process 306a corresponds to the processing of the acquisition units 151 and 251. Processing of the preprocessing process 306b corresponds to the processing of the preprocessing units 152 and 252. Processing of the generation process 306c corresponds to the processing of the generation units 153 and 253. Processing of the specifying process 306d corresponds to the processing of the specification units 154 and 254. Processing of the evaluation process 306e corresponds to the processing of the evaluation units 155 and 255.

Note that it is not necessarily needed for the hard disk device 307 to store each of the programs 307a to 307e beforehand. For example, each of the programs is stored in a "portable physical medium" such as a flexible disk (FD), a compact disc read only memory (CD-ROM), a digital versatile disc (DVD) disk, a magneto-optical disk, or an IC card to be inserted in the computer 300. Then, the computer 300 may read and execute each of the programs 307a to 307e.

## Claims

1. An evaluation method in which a computer executes processing comprising:
acquiring base sequence data;
generating a new piece of base sequence data by shifting positions, on the base sequence data, of a plurality of bases included in the acquired base sequence data;
specifying a partial base sequence that includes a base in which it is estimated that a genetic mutation is caused, from among a plurality of partial base sequences generated by dividing the plurality of bases included in the newly generated base sequence data from a reference position on the new piece of base sequence data according to a predetermined rule; and
evaluating the acquired base sequence data according to an appearance state in which an arrangement of the specified partial base sequence and a partial base sequence that has a predetermined positional relationship with the specified partial base sequence from among the plurality of partial base sequences appears in the plurality of partial base sequences generated by dividing a plurality of bases included in predetermined base sequence data from the reference position on the predetermined base sequence data according to the predetermined rule.

2. The evaluation method according to claim 1, wherein
the processing of generating the new piece of base sequence data generates the new piece of base sequence data by inserting a base into a partial base sequence that follows the partial base sequence at the reference position on the acquired base sequence data.

3. The evaluation method according to claim 1, wherein
the processing of generating the new piece of base sequence data generates the new piece of base sequence data by deleting a base in a partial base sequence that follows the partial base sequence at the reference position on the acquired base sequence data.

4. The evaluation method according to any one of claims 1 to 3, wherein
the processing of specifying the plurality of partial base sequences specifies a partial base sequence that includes a codon in which it is estimated that a genetic mutation is caused, from among a plurality of partial base sequences generated by performing division in codon units from the reference position of the new piece of base sequence data.

5. The evaluation method according to any one of claims 1 to 3, wherein
the processing of specifying the plurality of partial base sequences specifies a partial base sequence that includes a protein in which it is estimated that a genetic mutation is caused from among a plurality of partial base sequences generated by performing division in protein units from the reference position of the new piece of base sequence data.

6. The evaluation method according to any one of claims 1 to 3, wherein
the processing of evaluating compares the arrangement and a plurality of partial base sequences generated by dividing a plurality of bases included in predetermined base sequence data from the reference position on the predetermined base sequence data according to the predetermined rule and evaluates the number of continuously-matching partial base sequences.

7. An evaluation program in which a computer executes processing comprising:
acquiring base sequence data;
generating a new piece of base sequence data by shifting positions, on the base sequence data, of a plurality of bases included in the acquired base sequence data;
specifying a partial base sequence that includes a base in which it is estimated that a genetic mutation is caused, from among a plurality of partial base sequences generated by dividing the plurality of bases included in the newly generated base sequence data from a reference position on the new piece of base sequence data according to a predetermined rule; and
evaluating the acquired base sequence data according to an appearance state in which an arrangement of the specified partial base sequence and a partial base sequence that has a predetermined positional relationship with the specified partial base sequence from among the plurality of partial base sequences appears in the plurality of partial base sequences generated by dividing a plurality of bases included in predetermined base sequence data from the reference position on the predetermined base sequence data according to the predetermined rule.

8. The evaluation program according to claim 7, wherein
the processing of generating the new piece of base sequence data generates the new piece of base sequence data by inserting a base into a partial base sequence that follows the partial base sequence at the reference position on the acquired base sequence data.

9. The evaluation program according to claim 7, wherein
the processing of generating the new piece of base sequence data generates the new piece of base sequence data by deleting a base in a partial base sequence that follows the partial base sequence at the reference position on the acquired base sequence data.

10. The evaluation program according to any one of claims 7 to 9, wherein
the processing of specifying the plurality of partial base sequences specifies a partial base sequence that includes a codon in which it is estimated that a genetic mutation is caused, from among a plurality of partial base sequences generated by performing division in codon units from the reference position of the new piece of base sequence data.

11. The evaluation program according to any one of claims 7 to 9, wherein
the processing of specifying the plurality of partial base sequences specifies a partial base sequence that includes a protein in which it is estimated that a genetic mutation is caused from among a plurality of partial base sequences generated by performing division in protein units from the reference position of the new piece of base sequence data.

12. The evaluation program according to any one of claims 7 to 9, wherein
the processing of evaluating compares the arrangement and a plurality of partial base sequences generated by dividing a plurality of bases included in predetermined base sequence data from the reference position on the predetermined base sequence data according to the predetermined rule and evaluates the number of continuously-matching partial base sequences.

13. An evaluation device comprising:
an acquisition unit configured to acquire base sequence data;
a generation unit configured to generate a new piece of base sequence data by shifting positions, on the base sequence data, of a plurality of bases included in the acquired base sequence data;
a specification unit configured to specify a partial base sequence that includes a base in which it is estimated that a genetic mutation is caused, from among a plurality of partial base sequences generated by dividing the plurality of bases included in the newly generated base sequence data according to a predetermined rule from a reference position on the new piece of base sequence data; and
an evaluation unit configured to evaluate the acquired base sequence data according to an appearance state in which an arrangement of the specified partial base sequence and a partial base sequence that has a predetermined positional relationship with the specified partial base sequence from among the plurality of partial base sequences appears in the plurality of partial base sequences generated by dividing a plurality of bases included in predetermined base sequence data from the reference position on the predetermined base sequence data according to the predetermined rule.

14. The evaluation device according to claim 13, wherein
the generation unit is further configured to generate the new piece of base sequence data by inserting a base into a partial base sequence that follows the partial base sequence at the reference position on the acquired base sequence data.

15. The evaluation device according to claim 13, wherein
the generation unit is further configured to generate the new piece of base sequence data by deleting a base in a partial base sequence that follows the partial base sequence at the reference position on the acquired base sequence data.

16. The evaluation device according to any one of claims 13 to 15, wherein
the specification unit is further configured to specify a partial base sequence that includes a codon in which it is estimated that a genetic mutation is caused, from among a plurality of partial base sequences generated by performing division in codon units from the reference position of the new piece of base sequence data.

17. The evaluation device according to any one of claims 13 to 15, wherein
the specification unit is further configured to specify a partial base sequence that includes a protein in which it is estimated that a genetic mutation is caused from among a plurality of partial base sequences generated by performing division in protein units from the reference position of the new piece of base sequence data.

18. The evaluation device according to any one of claims 13 to 15, wherein
the evaluation unit configured to compare the arrangement and a plurality of partial base sequences generated by dividing a plurality of bases included in predetermined base sequence data from the reference position on the predetermined base sequence data according to the predetermined rule and evaluates the number of continuously-matching partial base sequences.
